# EUROPEAN PATENT APPLICATION

(11) **EP 3 590 911 A1**
(43) Date of publication of application: **08.01.2020**
(21) Application number: 18181390.8
(22) Date of filing: 03.07.2018
(51) Int. Cl.: C07B 59/00, C07F 15/00, C07K 7/00, A61K 51/08

(54) **REAGENT AND PROCESS FOR THE SITE-SPECIFIC DEOXYFLUORINATION OF PEPTIDES**

(30) Priority: 01.07.2018 EP 18181055
(71) Applicant: Studiengesellschaft Kohle MbH, 45470 Mülheim an der Ruhr (DE)
(72) Inventor: RITTER, Tobias, 45470 Muelheim (DE); RICKMEIER, Henrick Jens, 40227 Duesseldorf (DE)

(57) **Abstract**

The present invention refers to reagents and methods for preparing a peptide sequence having a [¹⁸F]fluoro-aromatic amino acid side which may be further substituted, in particular a 4-[¹⁸F]fluoro-phenylalanine side chain in peptide sequences, by chemoselective radio-deoxyfluorination of an aromatic amino acid residue, in particular a tyrosine residue using a traceless-activating group and the reagents used in said process.

## Description

The present invention refers to reagents and methods for preparing a peptide sequence having a [¹⁸F]fluoro-aromatic amino acid side which may be further substituted, in particular a 4-[¹⁸F]fluoro-phenylalanine side chain in peptide sequences, by chemoselective radio-deoxyfluorination of an aromatic amino acid residue, in particular a tyrosine residue using a traceless-activating group and the reagents used in said process. The replacement of only one hydrogen atom with [¹⁸F]fluoride results in minimal perturbation of the structure of the peptide, which is desirable in the labeling of tracer candidates.

Radiolabeled peptides are advantageous positron emission tomography (PET) tracers, and have been applied in preclinical target evaluation, noninvasive diagnosis of diseases, and in the study of biochemical processes. The wide-spread use of small-peptide tracers is due to the favorable prospects of achieving high binding affinities, rapid blood clearance, and rapid synthesis by automated solid phase peptide synthesis (SPPS) for initial optimization.The labeling of native peptides without affecting their properties is desirable and can be achieved using ¹¹C, ¹³N and ¹⁵O isotope labeled structures, however, the short half-lives of 20 minutes or less of these nuclei often prevents meaningful imaging. The 110 min half-life of ¹⁸F is more appropriate for PET imaging with peptides, but native peptides with fluorine substitution are unknown. The smallest perturbation to a native peptide with ¹⁸F is the displacement of a single hydrogen atom. Despite the development of several fluorination methods, none of the conventional or modern methods using [¹⁸F]fluoride have been successful in achieving this goal. The lack of a conceptual breakthrough in this area is rooted in the dense functionality of peptides that can result in undesired hydrogen bonding to fluoride and undesired side reactions of reagents or catalysts with the peptide. With available methods, peptide labeling with [¹⁸F]fluoride is restricted to the use of prosthetic groups, which can be very useful, but alter the properties of the peptide as a conceptual limitation.

Currently, clinically used radiolabeled peptides, such as the somastatin-targeting compounds edotreotide and DOTA-TATE often contain ligands that are useful to chelate radioactive metals for therapy and diagnosis. Despite their utility, the large polar ligands substantially alter the structure of the native peptide and can have substantial effects on the pharmacokinetic profile.

There are several examples of the introduction of smaller labeled prosthetic groups to peptides, which can be expected to alter the properties of the peptides to a smaller extent. For example, Hooker and Buchwald (J. Am. Chem. Soc. 2017, 139, 7152-7155) reported a two-step protocol to label unprotected peptides on cysteine with a 4-[¹¹C]cyano-phenyl substituent. Peptide labeling with fluorine-18 can be achieved successfully with prefunctionalized peptides that are able to capture [¹⁸F]fluoride under mild conditions by the formation of strong Si-F, B-F or Al-F bonds (Chemistry A European Journal 2017, 23, 15553-15577). Introduction of fluorine-18 via carbon-sulfur bond formation was shown by the Gouverneur group (J. Am. Chem. Soc. 2018, 140, 1572-1575): synthesis of the [¹⁸F]Umemoto reagent in 5% non-decay corrected radiochemical yield (RCY) enabled the subsequent direct labeling of unmodified peptides with fluorine-18 at cysteine or homocysteine residues with [¹⁸F]CF₃ to afford radiolabeled thioethers. The prosthetic group methods have been applied successfully for large molecules such as proteins, however, structural modification has a larger effect for smaller molecules, such as small peptides (J Nucl Med 1993, 34, 2253-2263). The first method achieving peptide labeling without structural modifications was reported by Långström, who labeled homocysteine with [¹¹C]Mel to afford a [¹¹C]methionine side chain (half-life of ¹¹C: 20 min) (Labelled Compd. Radiopharm. 1981, 18, 479-487).

Despite recent progress, there is still no method available that serves for replacing a single hydrogen atom with [¹⁸F]fluoride in a native peptide. Substitution of a "non-functional" hydrogen atom would be the least invasive scenario to introduce ¹⁸F into a peptide, as opposed to substitution of a hydrogen from an O-H, S-H, or N-H functionality.

With the present method, the inventors describe the first such method; wherein the only structural necessity for its successful implementation is the presence of a phenylalanine residue in the native peptide. In more detail, the present inventors report a method to introduce a 4-[¹⁸F]fluoro-phenylalanine side chain into peptide sequences, by chemoselective radio-deoxyfluorination of a tyrosine residue using a traceless-activating group. The replacement of only one hydrogen atom with [¹⁸F]fluoride results in minimal perturbation of the structure of the peptide, which is desirable in the labeling of tracer candidates.

The present invention is therefore directed a [P-AR¹(RuCp)-OH] complex having the general formula (I): 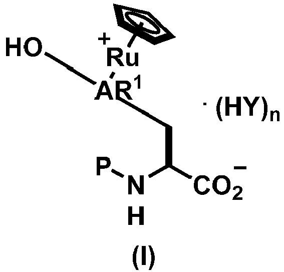
wherein AR¹ is an aromatic or heteroaromatic hydrocarbon having 5 to 14 carbon atoms, preferably six to ten carbon atoms, which may be further substituted by at least one C₁ to C₆ alkyl group and/or by at least one heteroatom,
wherein Y is an anion, preferably selected from CF₃CO₂⁻, triflate⁻ or OH⁻, n is 0 or 1, and P is a protective group, preferably a fluorenyl methoxy carbonyl (Fmoc), *tert*-butoxycarbonyl (Boc), or benzyloxycarbonyl (Cbz) group.

The present invention is furthermore directed to:
- a [P-AR¹(RuCp)-OH] complex having the general formula (I), wherein AR¹ is phenyl, 4-hydroxy-phenyl- or 1*H*-indol-3-yl, Y is an anion, preferably selected from CF₃CO₂⁻, triflate⁻ or OH⁻, n is 0 or 1, and P is a protective group, preferably a fluorenyl methoxy carbonyl (Fmoc), *tert*-butoxycarbonyl (Boc), or benzyloxycarbonyl (Cbz) group;
- a [AR¹(RuCp)-OH] complex having the general formula (IV): 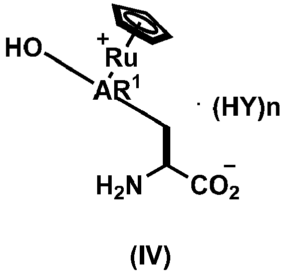 wherein AR¹ is an aromatic or heteroaromatic hydrocarbon having 5 to 14 carbon atoms, preferably six to ten carbon atoms, which may be further substituted by at least one C₁ to C₆ alkyl group and/or at least one heteroatom, Y is an anion, preferably selected from CF₃CO₂⁻, triflate⁻ or OH⁻, and n is 0 or 1;
- a [AR¹(RuCp)-OH] complex having the general formula (IV) as defined before, wherein AR¹ is phenyl; 4-hydroxy-phenyl- or 1*H*-indol-3-yl, Y is an anion, preferably selected from CF₃CO₂⁻, triflate⁻ or OH⁻, and n is 0 or 1;
- the use of the [P-AR¹⁻⁽RuCp)-OH]⁻complex (I) as defined above in a solid phase peptide synthesis, wherein AR¹ is an aromatic or heteroaromatic hydrocarbon having 5 to 14 carbon atoms, preferably six to ten carbon atoms, which may be further substituted by at least one C₁ to C₆ alkyl group or at least one heteroatom,
   wherein Y is an anion, preferably selected from CF₃CO₂⁻ , triflate⁻ or OH⁻, n is 0 or 1, and P is a protective group, preferably a fluorenyl methoxy carbonyl (Fmoc), *tert*-butoxycarbonyl (Boc), or benzyloxycarbonyl (Cbz) group;
- the use of the [P-AR¹⁻⁽RuCp)-OH]⁻complex (I) as defined above in a solid phase peptide synthesis, wherein AR¹ is phenyl; 4-hydroxy-phenyl- or 1*H*-indol-3-yl, Y is an anion, preferably selected from CF₃CO₂⁻ , triflate⁻ or OH⁻, and n is 0 or 1, and P is a protective group, preferably a fluorenyl methoxy carbonyl (Fmoc), *tert*-butoxycarbonyl (Boc), or benzyloxycarbonyl (Cbz) group;
- an oligo- or polypeptide having at least one amino acid residue of the formula (V) as defined above incorporated into the amino acid/peptide backbone, wherein AR¹ is an aromatic or heteroaromatic hydrocarbon having 5 to 14 carbon atoms, preferably six to ten carbon atoms, which may be further substituted by at least one C₁ to C₆ alkyl group or at least one heteroatom 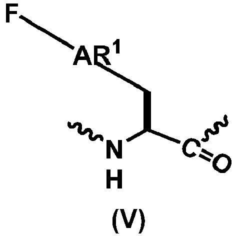
- an oligo- or polypeptide having at least one amino acid residue of the formula (V) in the amino acid/peptide backbone, wherein AR¹ is phenyl; 4-hydroxy-phenyl- or 1*H*-indol-3-yl; 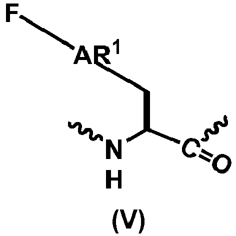
- the use of an oligo- or polypeptide as defined above for preparing a diagnostic composition for positron emission tomography (PET);
- the use of an [AR¹(RuCp)-OH]Y complex having the general formula (IV) for preparing a diagnostic composition, wherein AR¹ and Y have the meaning as defined above, and
- to a process for preparing a [AR¹(RuCp)-OH] complex (Ia) by reacting in a first step an aromatic amino acid (II) with a AR²⁻RuCp-complex (III) under UV-irradiation, 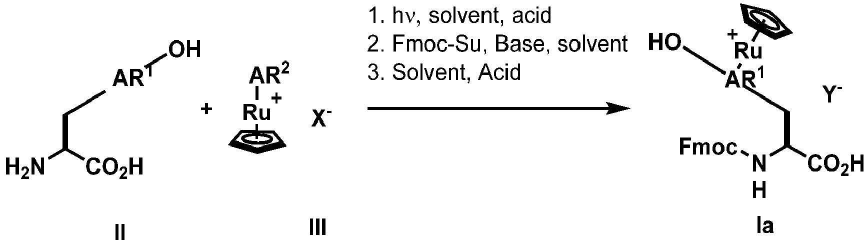
   wherein AR¹ is an aromatic or heteroaromatic hydrocarbon having 5 to 14 carbon atoms, preferably six to ten carbon atoms, which may be further substituted by at least one C₁ to C₆ alkyl group and/or at least one heteroatom, AR¹ preferably being phenyl, 4-hydroxy-phenyl- or 1*H*-indol-3-yl,
   wherein AR² is an aromatic or heteroaromatic hydrocarbon having 6 to 14 carbon atoms, preferably six to ten carbon atoms, which may be substituted by at least one C₁ to C₆ alkyl group and/or at least one hetero atom, AR² preferably being phenyl or naphthyl,
   wherein X⁻ is an anion, preferably selected from BF4⁻, CF₃SO₃⁻, and
   wherein Y⁻ is an anion selected from CF₃CO₂⁻, triflate⁻ or OH⁻, and, in a second step, introducing a Fmoc-protective group in the presence of a base under common conditions for introducing a Fmoc protective group into the obtained complex whereby a [FMOC-AR¹⁻⁽RuCp)-OH]⁻complex (I) is obtained, and, depending on the anion
   X⁻, acidifying the obtained solution.

Said [P-AR¹⁻⁽RuCp)-OH]⁻complex (I) can be used in a solid phase peptide synthesis to be coupled to an immobilized amino acid or oligo peptide, and after deprotecting by removing the Fmoc protective group and, optionally followed by further solid phase peptide synthesis steps (coupling Fmoc-amino acids, removing the protective group(s) the desired oligo- or polypeptide is obtained as generally illustrated in the following reaction scheme: 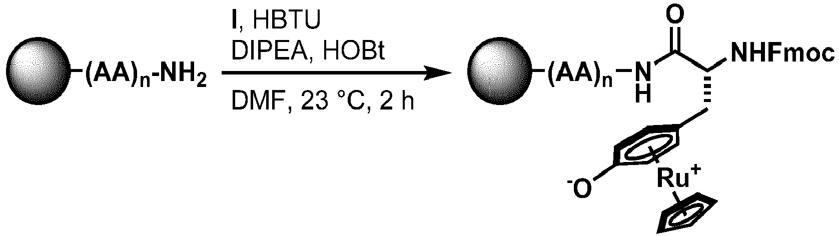 Wherein exemplarily:
HBTU is the coupling reagent 2-(1*H*-Benzotriazol-1-yl)-1,1,3,3-tetramethyluronium-hexafluorophosphate;
DIPEA is the Base
HOBt Hydroxybenzotriazol is auxiliary agent to prepare an activated triazole ester

The invention is also directed to the use of the [P-AR¹⁻⁽RuCp)-OH]⁻complex (I) in a solid phase peptide synthesis, to the so-obtained oligo- or polypeptide and to the process for fluorinating the so obtained oligo- or polypeptide to obtain a peptide with at least one amino acid having an [¹⁸F]fluoro substituent on the AR¹-side chain.

With the present invention, peptides comprising [¹⁸F]fluoro substituted tyrosine, phenylalanine, and tryptophan are easily available.

A method for fluorinating such peptides comprising the Ru-complex is provided by a process for preparing an oligo- or polypeptide having at least one amino acid residue of the formula (V) in the amino acid backbone, wherein AR¹ is phenyl; 4-hydroxy-phenyl- or 1*H*-indol-3-yl: 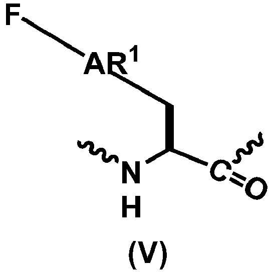 wherein an oligo- or polypeptide having at least one amino acid complex of the formula (IV), incorporated into the amino acid backbone, wherein AR¹, Y and n are as defined in claims 3 or 4, is reacted with a fluorine source in the presence of a imidazolium chloride and an oxalate compound, selected from bis(trimethylneopentylammonium) oxalate or Kryptand 2.2.2 and alkali oxalate, to fluorinate the AR¹ compound, optionally followed by acidification to remove protective groups, thereby providing an oligo- or polypeptide having a fluorinated AR¹compound as represented in the following scheme:

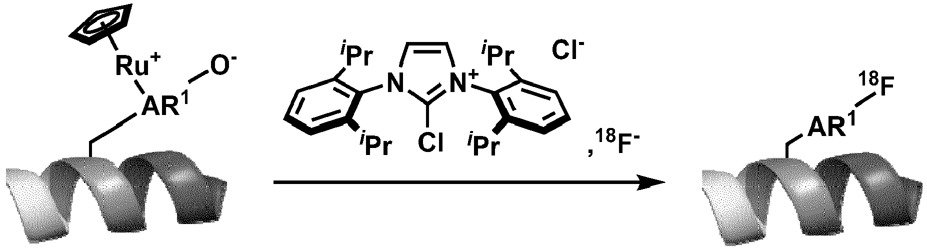

Said reaction may be carried out on an anion exchanger column loaded with 18F and elution with a preferably organic solvent, preferably a mixture of ethanol and pivalonitrile, at an elevated temperature of 100° to 150°C, in particular 125° to 135°C.

Said fluorination process is basically disclosed in EP17184127.3 which is incorporated herein by reference. The reaction conditions can be further improved on the basis of the teachings of said EP17184127.3.

In the sense of the invention, the definitions are to be understood as follows.

"Aryl" refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms provided in the aromatic ring system ("C₆₋₁₀ aryl"). In some embodiments, an aryl group has six ring carbon atoms ("C₆ aryl"; *e*.*g*., phenyl). In some embodiments, an aryl group has ten ring carbon atoms ("C₁₀ aryl"; *e*.*g*., naphthyl such as 1-naphthyl and 2-naphthyl). In some embodiments, an aryl group has fourteen ring carbon atoms ("C₁₄ aryl"; *e*.*g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i*.*e*., unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Heteroaryl" refers to a radical of a 5-14 membered monocyclic or bicyclic 4n+2 aromatic ring system (*e*.*g*., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e*.*g*., indolyl, quinolinyl, carbazolyl, and the like) the point of attachment can be on either ring, *i*.*e*., either the ring bearing a heteroatom (*e*.*g*., 2-indolyl) or the ring that does not contain a heteroatom (*e*.*g*., 5-indolyl).

In the invention, a heteroatom might be nitrogen, oxygen, sulfur, optionally being part of an hydrocarbon ring or optionally being bond to an alkyl group, preferably a C1 to C6 alkyl group, and for halogen.

As illustrated above and in the following experimental part, the inventors have developed the first method to label small peptides by replacing a single hydrogen, the para-hydrogen of phenylalanine or other aromatic or hetero aromatic amino acids, with [¹⁸F]fluoride by providing the present invention,. The inventive method is robust, tolerates all 20 canonical amino acids, can make use of conventional solid phase peptide synthesis, and labels at a single, pre-defined site due to the use of a traceless activating ruthenium group. While Ru-mediated deoxyfluorination of simple phenols has been developed previously, the inventive method represents a conceptual advance because it enables access to an important class of ¹⁸F-labeled molecules that has hitherto remained elusive.

The present invention is further illustrated by the following Figures and Examples:
The Figures serve for illustration purposes and show in:
- Figure 1:: Labeling approaches for peptide labeling.
- Figure 2:: Synthesis and Application of Novel Amino Acid Building Block
- Figure 2a:: Substrate Scope for Ruthenium-Mediated ¹⁸F-Deoxyfluorination of Peptides

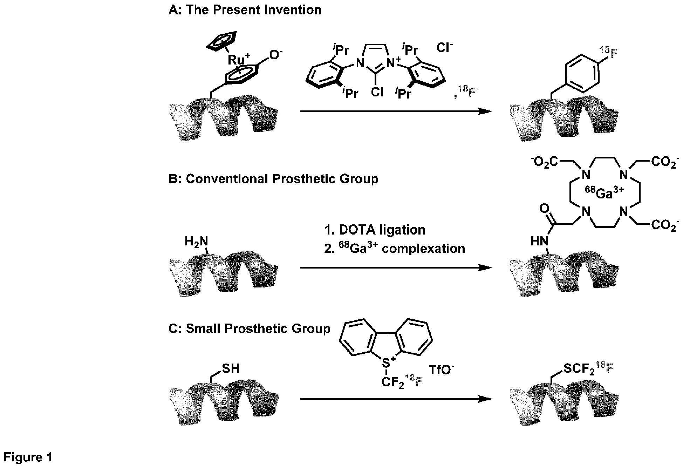

- Figure 1b: Reaction Optimization for Ruthenium-Mediated ¹⁸F-Deoxyfluorination of Peptides with reference to Table 1)

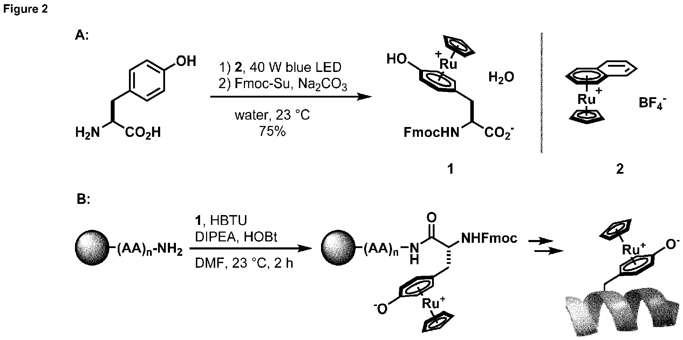

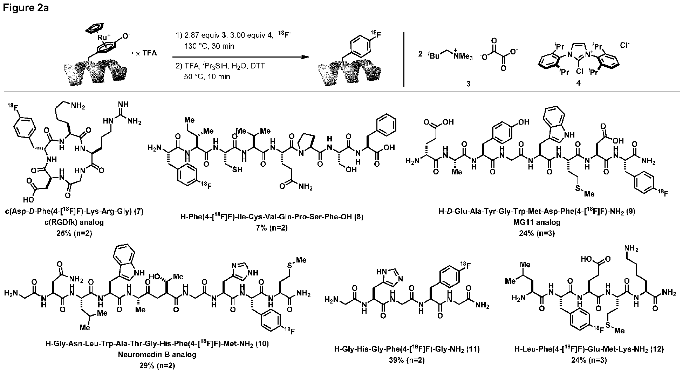

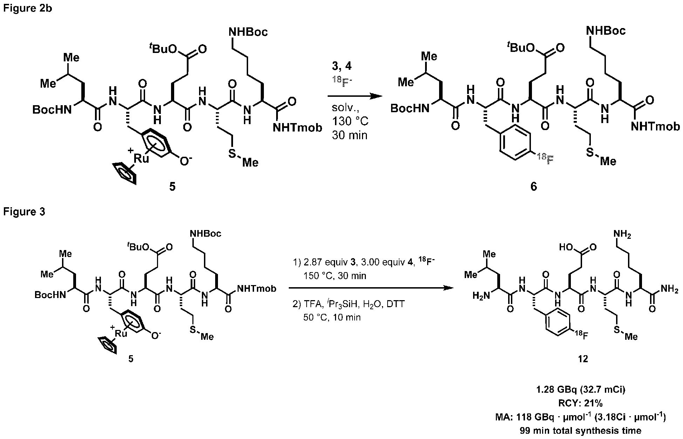

- Figure 2:: A Proposal for a Fully Automated Peptide Labeling Process

As illustrated in Figure 2, the non-natural, ruthenium-containing amino acid building block 1 can be prepared from tyrosine and commercially available ruthenium precursor 2 and can be purified on gram-scale without the need for chromatography (Figure 2A). Simple precipitation of the neutral zwitterion 1 provides analytically clean material that can participate readily in conventional solid-phase-peptide-synthesis (SPPS) using standard HBTU coupling. As a result, various peptides containing 1 can be accessed rapidly by modular synthesis to incorporate the ruthenium directing group (Figure 2B). The ruthenium and phenol functionality in 1 act as spectators in SPPS and did not engage in unwanted side reactions, such as ester bond formation at the phenolic group or undesired ruthenium leaching. The inert behavior may be a result of the tight η⁶ binding mode of the ruthenium to the π system of tyrosine, and the resulting lower nucleophilicity of the phenol. After SPPS on a 2-chlorotrityl resin using conventional protecting groups for amino acid building blocks, such as *tert*-butyloxycarbonyl (Boc) and fluorenylmethyloxycarbonyl (Fmoc) for lysine, the ruthenium-containing peptides were cleaved with a mixture of 20% hexafluoroisopropanol (HFIP) in DCM to afford the fully protected peptide. The peptide ruthenium complexes are stable towards air and moisture, preferably stored in their betain form with the complexed, deprotonated phenol moiety to balance the formal positive charge on the ruthenium(II) center, and can be purified by preparative high performance liquid chromatography (HPLC). When the peptide complexes were stored at -20 °C in a sealed vial, no degradation was observed for at least two months. Introduction of the ruthenium complex as part of an individually labeled amino acid building block during SPPS allows chemoselective, site-specific labeling of peptides as opposed to labeling of a native, assembled peptide. The ease and efficiency of SPPS mitigates the additional synthetic effort required to incorporate 1, and presents itself as an advantage in modularity and pace of peptide synthesis. The presence of additional phenylalanine, tyrosine, or tryptophan building blocks in the peptide does not pose a challenge in reactivity or site selectivity in the ensuing labeling reaction. Likewise, ruthenium migration from one side chain to another aromatic side chain has not been observed in any case of this study.

As illustrated in Figure 2a, the procedure is amenable to labeling complex small peptides that could potentially be used as PET tracers. Cyclic peptide **7** containing the Arg-Gly-Asp (RGD) motif, commonly used as a binding site in angiogenesis monitoring PET tracers, could be labeled in 25% decay corrected RCY after HPLC purification. The presence of a C-terminal free carboxylic acid during radiolabeling is tolerated, but tends to lower the yield. For example, octapeptide **8** was labeled in 7% RCY after HPLC purification. However, most peptide radiotracers contain C-terminal primary amides, which often mimics more closely the native peptide, and often have higher metabolic stability towards carboxypeptidases. The use of peptides containing C-terminal primary amides provide generally high yields, such as for octapeptide **9**, an analog of the gastrin releasing peptide (GRP)-receptor tracer MG11, which was isolated in 24% RCY after HPLC purification. The neuromedine B analog **10** containing 10 amino acids could be isolated after HPLC purification in 29% RCY. Pentapeptide **11** contains a histidine side chain that could function as internal base to result in epimerization;²⁷ synthesis of **11** was accomplished in 39% RCY after HPLC purification showed no epimerization through comparison with authentic samples of both diastereomers within the limits of detection (see SI). The generality of our protocol is highlighted by successful labeling of all substrates under identical reaction conditions. All 20 cononical amino acids were compatible with the protocol, and the ruthenium activation group can be positioned N-terminally, C-terminally, or within the core part of the peptide.

The results of the optimization tests for the reaction conditions for the reaction of **5** to **6** in the presence of **3** and **4** (Figure 2b) are given in Table 1.

| **Table 1 - Reaction Optimization^{a}** | | |
|---|---|---|
| Change of Reaction Conditions | Elution Efficiency [%]^{b} | RCC [%]^{c} |
| none | 81 | 84 |
| without oxalate 3 | 47 | 73 |
| Kryptofix, K₂C₂O₄ instead of 3 | 74 | 76 |
| 10 µL H₂O added | 57 | 78 |
| 2 mg of 5 instead of 7 mg | 67 | 44 |

| | | |
|---|---|---|
| ^{a} Reaction conditions: **5** (5.00 µmol), **3** (14.3 µmol), **4** (15.0 µmol) solv. = pivalonitrile:veratrole:ethanol (450 µL, 4:4:1, v:v:v), ^{b} determined by measuring the activity on the cartridge before and after elution ^{c} determined by radio-TLC and radio-HPLC (n = 2). | | |

As regards the results indicated in Table1, radiolabeling procedures with ¹⁸F typically begin with an aqueous elution of ¹⁸F fluoride trapped on an anion exchange cartridge, followed by azeotropic drying of the fluoride. In the context of this project, we have found a simple, useful method to avoid aqueous elution and azeotropic drying when elution was executed with the peptide ruthenium complex in a solvent mixture of ethanol and pivalonitrile directly. Key to successful elution was the use of bis(trimethylneopentylammonium) oxalate (**3**) (Figure 1, entry 1). The elution efficiency dropped from 81% with oxalate **3** to 47% if **3** was not added to the eluent. Alternatively a mixture of Kryptofix®222 and potassium oxalate can be used (elution efficiency: 74%). No special precautions towards air and moisture are required and just a small drop in radiochemical conversion (RCC) was observed if 10µL water were added to the eluent (Figure 1, entry 4). For typical experiments, we employed 5 µmol of peptide precursor, which corresponds to roughly 6-12 mg; lowering the amount to 1.5 µmol (ca. 2 mg) resulted in only a small drop of the RCC (44%) and may thus be acceptable if the peptide is precious. The ideal reaction temperature was determined to be 130 °C. Initially, we were concerned about the high temperature, however, for all the peptides evaluated, extensive decomposition was not an issue during the 30 minutes time required for the reaction. The low degradation could be due to the lack of acid or base additives that are able to catalyze epimerization of stereocenters, and the degradation of the product. Lowering the temperature or shortening the reaction time resulted in a different major product, likely the ruthenium-complexed ¹⁸F-labeled peptide, which, if formed, can be readily separated by HPLC.

In a typical experiment, [¹⁸F]fluoride is trapped from an aqueous solution on a quaternary methyl ammonium (QMA) cartridge, followed by removal of residual water by pushing 1 ml of acetonitrile through the QMA cartridge. The [¹⁸F]fluoride is eluted from the cartridge with a solution of the peptide complex, oxalate **3**, and chloroimidazolium chloride **4**. Then the cartridge is eluted sequentially with a mixture of veratrole and pivalonitrile into the same vial. The vial, containing 450 µL of reaction mixture, was sealed and heated for 30 min at 130 °C to afford the ¹⁸F-labeled, fully protected peptide. After concentration of the reaction mixture to dryness, the acid-labile protecting groups were removed with a mixture of trifluoroacetic acid (TFA, 437 µL), *DL*-dithiothreitol (DTT, 25 mg), water (25 µL), and triisopropylsilane (TIPS, 13 µL). The resulting deprotected peptides were purified by HPLC.

Initial attempts to automate the process on the cassette-based radiochemical synthesizer Elixys FLEX/CHEM connected to a PURE/FORM purification and formulation unit (Sofie Biosciences) were low yielding. To obtain the same results as in the manual reaction, the temperature had to be raised to 150 °C, possibly an artefact of a temperature difference between set and actual temperature. Starting from 11.4 GBq (308 mCi) of aqueous [¹⁸F]fluoride, the inventors were able to isolate 1.28 GBq (32.7 mCi) of HPLC-purified and peptide **12** within 99 min in 21% decay corrected radiochemical yield, formulated in ethanolic saline, as illustrated in Figure 3. Determination of the molar activity in this project is important because, while no-carrier-added [¹⁸F] fluoride is used, conventional SPPS employs reagents with PF₆⁻ in large excess that could, in principle, substantially dilute the molar activity. Gratifyingly, the molar activity of peptide **12** was 118 GBq · µmol⁻¹ (3.18 Ci · µmol⁻¹).

By the present invention, the first protocol is reported by the inventors to directly label peptides with [¹⁸F]fluoride by replacing a simple, virtually innocent hydrogen atom, which should result in only minimal perturbation of the properties of the corresponding native peptide. The inventors anticipate that their method will accelerate the development of novel peptide tracers due to its efficiency, predictability, and robustness.

The present invention is further illustrated by the following experimental part, including the applied methods and Examples.

### Examples

All air- and moisture-insensitive reactions were carried out under an ambient atmosphere and monitored by thin-layer chromatography (TLC) or liquid chromatography-coupled mass spectrometry (LC-MS). High-resolution mass spectra were obtained using Q Exactive Plus from Thermo. Concentration under reduced pressure was performed by rotary evaporation at 23-40 °C at an appropriate pressure. Purified compounds were further dried under vacuum (0.01-0.5 mbar). Yields refer to purified and spectroscopically pure compounds. All air- and moisture-sensitive manipulations were performed using oven-dried glassware (130 °C for a minimum of 12 hours), including standard Schlenk and glove-box techniques under an atmosphere of argon, although they can operate equally under an atmosphere of nitrogen.¹ Irradiation of photochemical reactions were carried out using a 40 W blue LED (Kessil A 160WE Tuna Blue).

### Solvents

Acetonitrile, dichloromethane, and methanol were purchased from Sigma-Aldrich and used as received. Peptide grade dimethylformamide (DMF) and piperidine were purchased from Iris-Biotech and used as received. Ethanol (≥99.8%) was purchased from Honeywell and used as received. Trimethylacetonitrile (98+%) was purchased from Alfa Aesar and used as received. Anhydrous solvents were obtained from Phoenix Solvent Drying Systems. All deuterated solvents were purchased from Euriso-Top.

### Chromatography

Thin layer chromatography (TLC) was performed using EMD TLC plates pre-coated with 250 µm thickness silica gel 60 F₂₅₄ plates and visualized by fluorescence quenching under UV light. Flash chromatography was performed using silica gel (40-63 µm particle size) purchased from Geduran. Preparatory high-performance liquid chromatographic separation was executed on Shimadzu Prominence Preparative HPLC system.

### Spectroscopy and Instruments

NMR spectra were recorded on a Bruker Ascend™ 500 spectrometer operating at 500 MHz, 126 MHz, and 471 MHz, and for ¹H, ¹³C, and ¹⁹F acquisitions, respectively. Chemical shifts are reported in ppm with the solvent resonance as the internal standard. For ¹H NMR: CDCl₃, δ 7.26; CD₃OD, δ 3.31; (CD₃)₂SO, δ 2.50; CD₃CN, δ 1.94. For ¹³C NMR: CDCl₃, δ 77.16; CD₃OD, δ 49.00; (CD₃)₂SO, δ 39.52; CD₃CN, δ 1.32.² 19F NMR spectra were referenced using a unified chemical shift scale based on the 1H resonance of tetramethylsilane (1% v:v solution in the respective solvent).³ Data is reported as follows: s = singlet, d = doublet, t = triplet, q = quartet, m = multiplet, br = broad; coupling constants in Hz; integration. Liquid chromatography-coupled mass spectroscopic data were obtained on Agilent 1260 Infinity Automated LC/MS Purification System.

### Bis(cyclopentadienyl)ruthenium(II) (S1)

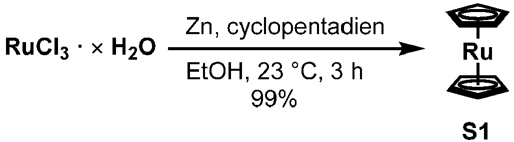

A two-neck round-bottom flask (500 mL) equipped with a Teflon-coated magnetic stirring bar and a thermometer, was charged with ruthenium trichloride hydrate (RuCl₃ × H₂O, 12.3 g, 47 mmol, 1.0 equiv) and absolute ethanol (140 mL, c = 0.34 M). The reaction flask was placed in an ice bath, and the reaction mixture was cooled to 0 °C, then cyclopentadiene (39 mL, 31 g, 0.47 mmol, 10 equiv)⁴ was added via syringe to the dark red solution. Zinc dust (∼325 mesh, 99.9% (metals basis), 31 g, 0.47 mmol, 10 equiv) was added over 60 minutes in 10 portions to the stirred solution, and the temperature was kept between 0 °C and 10 °C during the addition. The reaction mixture was stirred at 0 °C for 30 minutes, then the ice bath was removed, and stirring was continued for 3 hours. The suspension was filtered over a 60 mL Buchner funnel with micro porosity (code M), and the metallic grey solid was washed with hot toluene (100 °C, 4 × 140 mL). The filtrate was concentrated on a rotary evaporation to dryness, and the brown residue was then dissolved in toluene (520 mL) at 23 °C and passed through a plug of silica gel (20 g), which was subsequently rinsed with toluene (280 mL). The resulting yellowish solution was concentrated *in vacuo* to dryness to afford ruthenocene (**S1**) as pale yellow solid (10.8 g, 46.7 mmol, 99% yield).
Note: Ruthenium(III) chloride solid (metal content: 38.0%-41.0% ruthenium) was purchased from Johnson Matthey and used without purification.
**Melting point:**⁵ 199 °C.
**HRMS-EI (m/z)** calc'd for C₁₀H₁₀Ru [M]⁺, 231.981967; found, 231.982042; deviation: 0.32 ppm.

### [(Cp)Ru(η⁶-naphthalene)]·BF₄ (2)

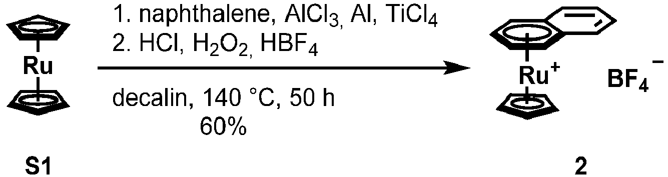

Under inert atmosphere, an oven-dried two neck round-bottom flask (500 mL) equipped with a reflux condenser and a Teflon-coated egg-shaped magnetic stirring bar was charged with ruthenocene (**S1**) (4.52 g, 19.5 mmol, 1.00 equiv), naphthalene (25.0 g, 195 mmol, 10.0 equiv), AlCl₃ (2.61 g, 19.5 mmol, 1.00 equiv), and aluminum powder (∼325 mesh, 99.7%, 264 mg, 9.77 mmol, 0.500 equiv). Then dry decalin (0.13 L, c = 0.15 M) was added, followed by dropwise addition of TiCl₄ (1.07 mL, 1.85 g, 9.77 mmol, 0.500 equiv) via a syringe. The resulting red suspension was heated to 140 °C and was then stirred for 50 hours at 140 °C. The oil bath was removed, and after cooling to room temperature, the reaction mixture was poured onto a mixture of ice (150 g), aqueous concentrated HCl-solution (28 mL), and H₂O₂ (35% solution in H₂O, 28 mL). The aqueous layer was separated from the organic layer with the aid of a separatory funnel, and the aqueous layer was washed with pentane (2 × 100 mL). The combined organic layers were extracted with water (50 mL), and then fluoroboric acid (48% solution in water, 5.1 mL, 7.2 g, 39 mmol, 2.0 equiv) was added to the combined aqueous layers in the reaction flask. The resulting orange solution was stirred for 15 minutes, and then the suspension was extracted with dichloromethane (4 × 100 mL). The combined organic layers were dried over sodium sulfate, filtered, and concentrated by rotary evaporation to dryness. The brown residue was dissolved in dichloromethane (15 mL) and then added dropwise through a syringe-filter to vigorously stirred diethyl ether (150 mL). The suspension was filtered through a 60 mL Buchner funnel with micro porosity (code M), and the pale yellow solid was washed with diethyl ether (2 × 30 mL), and dried *in vacuo* to afford **2** as a yellow solid (4.50 g, 11.8 mmol, 60% yield).
**HRMS-EI (m/z)** calc'd for C₁₅H₁₃Ru [M-BF₄]⁺, 295.00553; found, 295.00563; deviation: 0.35 ppm.

### [Fmoc-tyrosine(RuCp)-O]·H₂O (1)

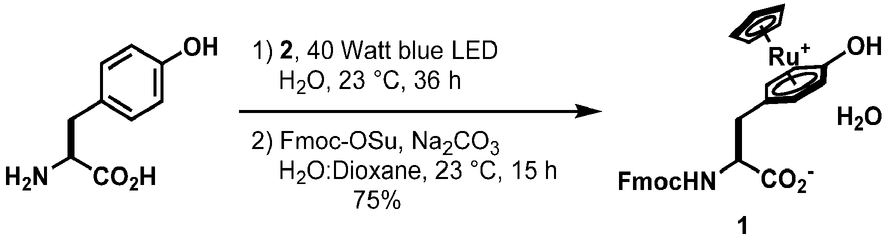

A round-bottom flask (250 mL) equipped with a Teflon-coated magnetic stirring bar was charged with [(Cp)Ru(η⁶-naphthalene)]·BF₄ (**2**) (4.61 g, 12.1 mmol, 1.10 equiv), *L*-tyrosine (1.99 g, 11.0 mmol, 1.00 equiv), water (0.11 L, c = 0.10 M), and fluoroboric acid (48% in water, 2.9 mL, 4.0 g, 22 mmol, 2.0 equiv). The yellow suspension was irradiated for 36 h with blue LED light (Kessil A 160WE Tuna Blue, 40 W). It is crucial that the tyrosine is fully consumed at this point, because if not the product must be purified by column chromatography. If required reaction time needs to be adjusted and more ruthenium precursor needs to be added. The resulting beige suspension was basified with sodium carbonate (3.85 g, 36.3 mmol, 3.30 equiv). The suspension was cooled to 0 °C with an ice bath, and then a solution of Fmoc-OSu (4.45 g, 13.2 mmol, 1.20 equiv) in dioxane (55 mL) was added. The reaction mixture was stirred at 0 °C for 1 hour, then at 23 °C for 14 hours. The solution was concentrated by rotary evaporation to two-thirds of the original volume, and then the aqueous layer was washed with dichloromethane (2 × 30 mL). The aqueous layer was acidified with HCI (4 M solution in H₂O) to pH 4, and was then extracted with DCM (3 × 100 mL). The combined organic layers were dried over sodium sulfate, filtered, and concentrated by rotary evaporation to dryness. The brown residue was dissolved in dichloromethane (15 mL) and filtered through a 15 mL Buchner funnel with fine porosity (code f) into vigorously stirred acetonitrile (150 mL). The suspension was filtered over a 60 mL Buchner funnel with micro porosity (code M), and the beige solid was washed with acetonitrile (2 × 10 mL), and dried *in vacuo* to dryness to afford **1** as a beige solid (4.83 g, 8.24 mmol, 75% yield).
**R_{f} =** 0.26 (EtOAc:MeCN:TFA, 9:1:0.1, v:v:v).
**R_{f} =** 0.56 (EtOAc:MeCN:TFA, 8:2:0.1, v:v:v).
**HRMS-ESI (m/z)** calc'd for C₂₉H₂₆NO₅Ru [M+H]⁺,570.08490; found, 570.085020; deviation: 0.21 ppm.
**Elemental Analysis** calc'd for C₂₉H₂₇NO₆Ru: C, 59.38; H, 4.64; found: C, 59.06; H, 4.31.

### [(Cp)Ru(η⁶-naphthalene)]·CF₃SO₃ (S2)

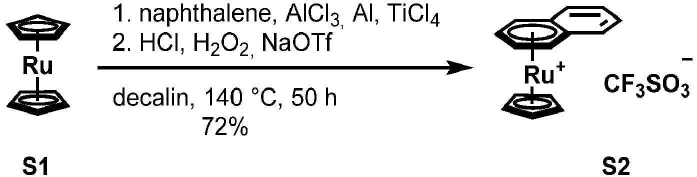

Under inert atmosphere, an oven-dried two neck round-bottom flask (1000 mL) equipped with a reflux condenser and a Teflon-coated egg-shaped magnetic stirring bar was charged with ruthenocene (**S1**) (10.7 g, 46.3 mmol, 1.00 equiv), naphthalene (59.3 g, 0.463 mol, 10.0 equiv), AlCl₃ (6.17 g, 46.3 mmol, 1.00 equiv), and aluminum powder (∼325 mesh, 99.7%, 624 mg, 23.1 mmol, 0.500 equiv). Then dry decalin (0.25 L, c = 0.19 M) was added, followed by dropwise addition of TiCl₄ (2.53 mL, 4.39 g, 23.1 mmol, 0.500 equiv) via a syringe. The resulting red suspension was heated to 140 °C and was then stirred for 50 hours at 140 °C. The oil bath was removed, and after cooling to room temperature, the reaction mixture was poured onto a mixture of ice (300 g), aqueous concentrated HCl-solution (66 mL), and H₂O₂ (50% solution in H₂O, 46 mL). The aqueous layer was separated from the organic layer with the aid of a separatory funnel, and the aqueous layer was washed with pentane (2 × 100 mL). The combined organic layers were extracted with water (50 mL), and then sodium triflate (15.9 g, 92.5 mmol, 2.00 equiv) was added to the combined aqueous layers in the reaction flask. The resulting orange solution was stirred for 15 minutes, and then the suspension was extracted with dichloromethane (5 × 240 mL). The combined organic layers were dried over sodium sulfate, filtered, and concentrated by rotary evaporation to dryness. The brown residue was dissolved in dichloromethane (30 mL) and then added dropwise through a syringe-filter to vigorously stirred diethyl ether (300 mL). The suspension was filtered through a 60 mL Büchner funnel with micro porosity (code M), and the pale yellow solid was washed with diethyl ether (2 × 30 mL), and dried *in vacuo* to afford **S2** as a yellow solid (15.1 g, 34.1 mmol, 72% yield).
**Melting point:** 112 °C.
**HRMS-EI (m/z)** calc'd for C₁₅H₁₃Ru [M-OTf]⁺, 295.00553; found, 295.00563; deviation: 0.35 ppm.
**Elemental Analysis** calc'd for C₁₆H₁₃F₃O₃RuS: C, 43.34; H, 2.96; found: C, 43.31; H, 2.94. **UV-vis Spectroscopy** (H₂O, 23°C): 360 nm (ε = 673 M⁻¹ · cm⁻¹).

### [Fmoc-tyrosine(RuCp)-OH]·CF₃CO₂ (S3)

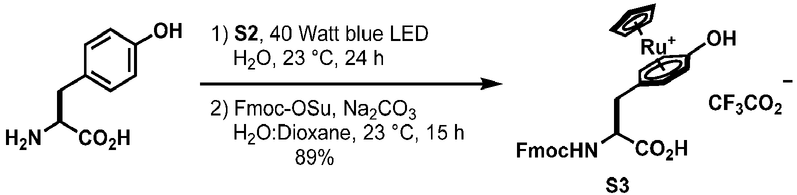

A round-bottom flask (2 L) equipped with a Teflon-coated magnetic stirring bar was charged with [(Cp)Ru(η⁶-naphthalene)]·CF₃SO₃ (**S2**) (6.65 g, 15.0 mmol, 1.00 equiv), *L*-tyrosine (3.40 g, 18.8 mmol, 1.25 equiv), water (0.75 L, c = 20 M), and trifluoroacetic acid (2.87 mL, 4.27 g, 37.5 mmol, 2.50 equiv). The yellow suspension was irradiated for 24 h with blue LED light (Kessil A 160WE Tuna Blue, 40 W), then the resulting beige suspension was extracted with hexane (2 × 200 mL), and the combined organic layers were extracted with water (50 mL). The combined aqueous layers were basified with Na₂CO₃ (7.95 g, 75.0 mmol, 5.00 equiv) and dioxane (0.25 L) was added. The suspension was cooled to 0 °C with an ice bath, and then a solution of Fmoc-OSu (7.59 g, 22.5 mmol, 1.50 equiv) in dioxane (0.25 L) was added. The reaction mixture was stirred at 0 °C for 1 hour, then at 23 °C for 14 hours. The solution was concentrated by rotary evaporation to half the original volume, and then the aqueous layer was washed with dichloromethane (3 × 200 mL). The aqueous layer was acidified with trifluoroacetic acid to pH 3 and then extracted with dichloromethane (4 × 300 mL). The combined organic layers were dried over sodium sulfate, filtered, and concentrated *in vacuo* to dryness. The residual brown solid was purified by column chromatography (EtOAc:MeCN:TFA, 90:9:1, v:v:v) to afford **S3** as a yellow powder (9.15 g, 13.4 mmol, 89% yield).
**R_{f} =** 0.26 (EtOAc:MeCN:TFA, 9:1:0.1, v:v:v).
**R_{f} =** 0.56 (EtOAc:MeCN:TFA, 8:2:0.1, v:v:v).
**HRMS-ESI (m/z)** calc'd for C₂₉H₂₆NO₅Ru [M-CF₃CO₂]⁺,570.08490; found, 570.08505; deviation: 0.26 ppm.

### [Fmoc-D-tyrosine(RuCp)-OH]·CF₃CO₂ (S4)

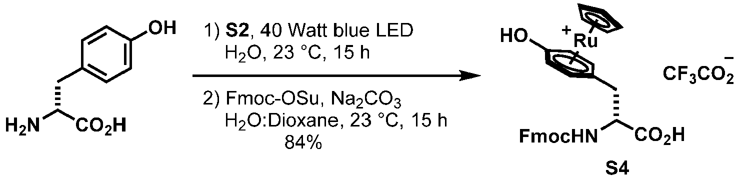

A round-bottom flask (1 L) equipped with a Teflon-coated magnetic stirring bar was charged with [(Cp)Ru(η⁶-naphthalene)]·CF₃SO₃ (**S2**) (3.00 g, 6.77 mmol, 1.00 equiv), *D*-tyrosine (1.35 g, 7.44 mmol, 1.10 equiv), water (0.27 L, c = 25 mM), and hydrochloric acid (37% in water, 1.1 mL, 1.3 g, 7.4 mmol, 2.0 equiv). The yellow suspension was irradiated for 15 h with blue LED light (Kessil A 160WE Tuna Blue, 40 W), then the resulting beige suspension was extracted with hexane (2 × 120 mL), and the combined organic layers were extracted with water (50 mL). The combined aqueous layers were basified with Na₂CO₃ (2.15 g, 20.3 mmol, 3.00 equiv) and dioxane (0.12 L) was added. The suspension was cooled to 0 °C with an ice bath and then a solution of Fmoc-OSu (2.74 g, 8.12 mmol, 1.20 equiv) in dioxane (0.12 L) was added. The reaction mixture was stirred at 0 °C for 1 hour, then at 23 °C for 14 hours. The solution was concentrated by rotary evaporation to half the original volume, and then the aqueous layer was washed with dichloromethane (3 × 120 mL). The aqueous layer was acidified with trifluoroacetic acid to pH 3 and then extracted with dichloromethane (4 × 120 mL). The combined organic layers were dried over sodium sulfate, filtered, and concentrated *in vacuo* to dryness. The residual beige solid was purified by column chromatography (EtOAc:MeCN:TFA, 90:9:1 v:v:v) to afford **S4** as a yellow powder (3.89 g, 5.70 mmol, 84% yield).
**R_{f}** = 0.26 (EtOAc:MeCN:TFA, 9:1:0.1, v:v:v).
**R_{f} =** 0.56 (EtOAc:MeCN:TFA, 8:2:0.1, v:v:v).
**HRMS-ESI (m/z)** calc'd for C₂₉H₂₆NO₅Ru [M-CF₃CO₂]⁺,570.08490; found, 570.085070; deviation: 0.30 ppm.

### Neopentyltrimethylammonium iodide (S5)

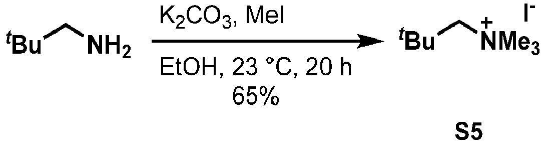

A round-bottom flask (250 mL) equipped with a Teflon-coated magnetic stirring bar was charged with neopentylamine (7.04 mL, 5.25 g, 60.3 mmol, 1.00 equiv) and ethanol (95 mL, c = 0.63 M). Potassium carbonate (11.0 g, 79.6 mmol, 1.32 equiv) and methyl iodide (12.0 mL, 27.3 g, 193 mmol, 3.20 equiv) were added sequentially to the reaction mixture, and then the reaction mixture was stirred at 23 °C for 20 h. The suspension was filtered through a 60 mL Büchner funnel with micro porosity (code M), and the filtrate was concentrated *in vacuo* to dryness to give a yellow solid. Recrystallization from isopropanol (200 mL) afforded **S5** as a colorless crystalline solid (10.1 g, 39.2 mmol, 65% yield).
**HRMS-ESI (m/z)** calc'd for C₈H₂₀N [M-I]⁺, 130.159024; found,130.159010; deviation: 0.11 ppm.

### Bis(neopentyltrimethylammonium) oxalate (3)

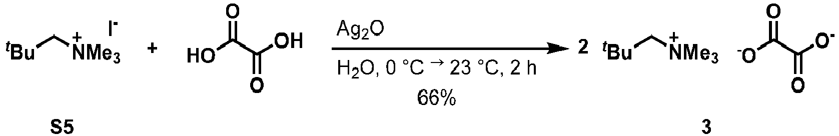

A round-bottom flask (100 mL) equipped with a Teflon-coated magnetic stirring bar and a thermometer was charged with neopentyltrimethylammonium iodide (**S5**) (3.00 g, 11.7 mmol, 1.00 equiv) and water (21 mL, c = 0.55 M). The reaction flask was placed in an ice bath, and the reaction mixture was cooled to 0 °C then silver(I) oxide (1.49 g, 6.42 mmol, 0.550 equiv) was added. The suspension was allowed to warm to 23 °C. After 1.5 h, oxalic acid (578 mg, 6.42 mmol, 0.550 equiv) was added, and the reaction mixture was stirred for 30 minutes. The suspension was filtered over a 60 mL Büchner funnel with micro porosity (code M), and the residual product was extracted with water (3 × 30 mL). The filtrate was concentrated by rotary evaporation to afford an oily residue. The oil was dissolved in acetonitrile (20 mL), filtered, and recrystallized by vapor diffusion with diethyl ether (20 mL). The colorless crystalline solid (**3**) was collected by filtration and dried *in vacuo* for 15 h at 75 °C (1.34 g, 3.85 mmol, 66%).
Note: Compound **3** is hygroscopic and needs to be stored in a closed vial or in a desiccator.

### General procedure for peptide synthesis.

Peptides were synthesized by solid-phase peptide synthesis using the Fmoc/*t*Bu-orthogonal strategy on a 2-chlorotritly chloride resin (100-200 mesh, 1% DVB, 1.6 mmol·g⁻¹) or a Fmoc-Rink-Amid-2CT resin (200-400 mesh, 1% DVB, 0.68 mmol·g⁻¹).¹⁰

### General loading procedure (2-CTC resin):

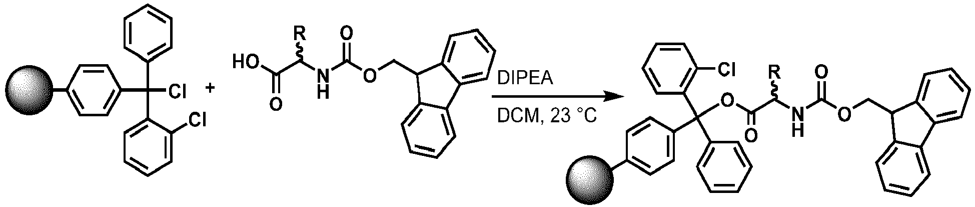

A peptide synthesis vessel was charged with 2-chlorotrityl chloride resin and DCM (30 mL · g⁻¹ resin). The suspension was shaken with the aid of a Heidolph Vibramax 100 for 30 min at 23 °C. The liquid was removed via vacuum filtration, and a solution of Fmoc-protected amino acid (4.00 equiv) and DIPEA (10.0 equiv) in DCM (30 mL · g⁻¹ resin) was added into the peptide synthesis vessel. The resulting suspension was shaken for 15 hours at 23 °C, and then the liquid was removed via vacuum filtration. The resin was washed with DCM (3 × 20 mL · g⁻¹ resin × 2 min), and a solution of DIPEA, MeOH, and DCM (1:2:17, v:v:v, 30 mL · g⁻¹ resin) was added into the peptide synthesis vessel. The suspension was shaken for 1 hour at 23 °C, and then the resin was washed sequentially with DMF (2 × 20 mL · g⁻¹ resin), DCM (2 × 20 mL · g⁻¹ resin), MeOH (2 × 20 mL · g⁻¹ resin), and Et₂O (2 × 20 mL · g⁻¹ resin). The resin was dried under vacuum, and the loading efficiency was determined by UV-vis spectroscopy at 289.8 nm.⁹ **General washing procedure:** Into the peptide synthesis vessel containing resin was added the stated washing-solvent (20 mL · g⁻¹ resin). The suspension was shaken for 2 minutes at 23 °C, and then the liquid was removed via vacuum filtration.

### General deprotection procedure:

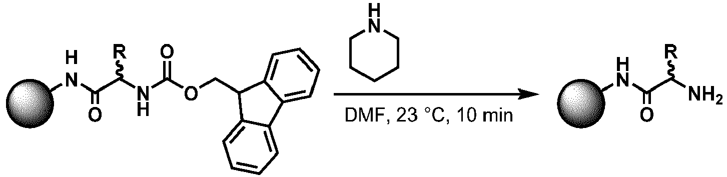

Into the peptide synthesis vessel containing resin-bound Fmoc-protected peptide was added 20% piperidine in DMF (v:v, 20 mL · g⁻¹ resin), and the suspension was shaken for 5 minutes at 23 °C. Then the liquid was removed via vacuum filtration. This deprotection sequence was repeated once, and then the resin was washed with DMF (3 × 20 mL · g⁻¹ resin× 2 min).

### General HBTU/HOBt coupling procedure:

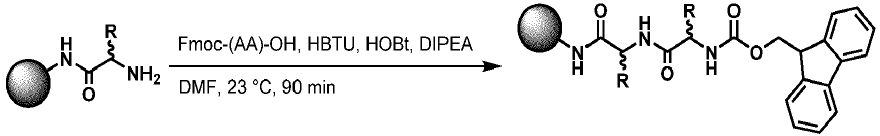

A round-bottom flask equipped with a Teflon-coated magnetic stirring bar was charged with Fmoc-protected amino acid (Fmoc-(AA)-OH, 4.00 equiv), HBTU (3.90 equiv), HOBt hydrate (3.90 equiv), DIPEA (8.00 equiv), and DMF (10 mL · g⁻¹ resin). The solution was stirred for 15 minutes at 23 °C and was then added into the peptide synthesis vessel. The vessel was shaken for 90 minutes at 23 °C, and then the liquid was removed via vacuum filtration. The resin was washed with DMF (3 × 10 mL·g⁻¹ resin × 2 min).

### General [Fmoc-Tyr(RuCp)-OH]·CF₃CO₂ coupling procedure:

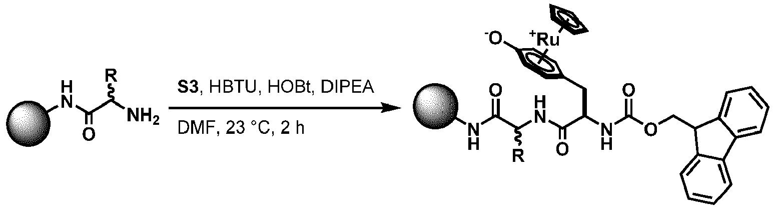

A round-bottom flask equipped with a Teflon-coated magnetic stirring bar was charged with [Fmoc-Tyr(RuCp)-OH]·CF₃CO₂ (**S3**) (2.00 equiv), HBTU (1.90 equiv), HOBt hydrate (1.90 equiv), DIPEA (16.0 equiv), and DMF (10 mL·g⁻¹ resin). The solution was stirred for 1 minute at 23 °C and was then added into the peptide synthesis vessel. The vessel was shaken for 2 h at 23 °C, and then the liquid was removed via vacuum filtration. The resin was washed with DMF (3 × 10 mL · g⁻¹ resin × 2 min).

### General [Fmoc-Tyr(RuCp)-O]·H₂O coupling procedure:

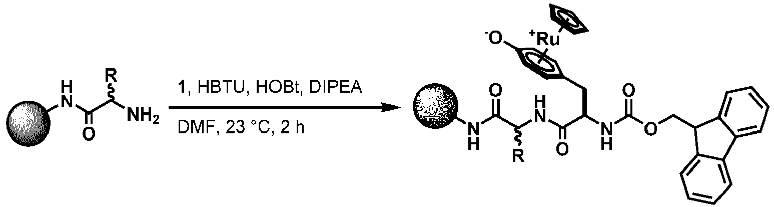

A round-bottom flask equipped with a Teflon-coated magnetic stirring bar was charged with [Fmoc-Tyr(RuCp)-O]·H₂O (**1**) (2.00 equiv), HBTU (1.90 equiv), HOBt hydrate (1.90 equiv), DIPEA (16.0 equiv), and DMF (10 mL·g⁻¹ resin). The solution was stirred for 1 minute at 23 °C, and was then added into the peptide synthesis vessel. The vessel was shaken for 2 h at 23 °C, and then the liquid was removed via vacuum filtration. The resin was washed with DMF (3 × 10 mL · g⁻¹ resin × 2 min).

### General Boc protection procedure:

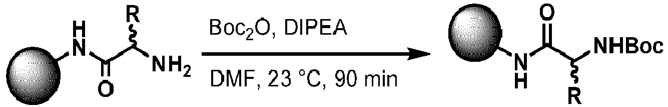

To the resin bound peptide was added a solution of di-*tert*-butyldicarbonate (Boc₂O) (4.00 equiv) and DIPEA (8.00 equiv) in DMF (20.0 mL · g⁻¹ resin), then the peptide synthesis vessel was shaken for 2 hours at 23 °C. The liquid was removed via vacuum filtration, and the resin was washed with DMF (3 × 20 mL · g⁻¹ × 2 min).

### General cleavage conditions:

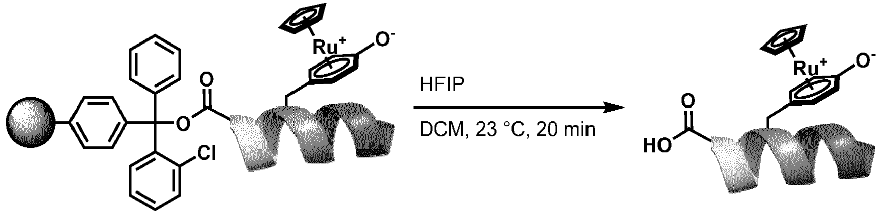

The resin was washed with DCM (3 × 20 mL · g⁻¹ resin × 2 min). Then a solution of 20% of hexafluoroisopropanol (HFIP) in DCM (v:v) (50 mL · g⁻¹ resin) was added to the resin, and the suspension was shaken for 20 minutes at 23 °C. The liquid was collected via vacuum filtration, and a solution of 20% of HFIP in DCM (v:v, 50 mL · g⁻¹ resin) was added to the resin, and the suspension was shaken for 50 minutes at 23 °C. The liquid was collected via vacuum filtration, and the combined organic layers were concentrated *in vacuo* to dryness and were analyzed via LC-MS.

### [H-Asp(^{t}Bu)-D-Phe(4-F)-Lys(Boc)-Arg(Pbf)-Gly-OH]·CF₃CO₂H (S6)

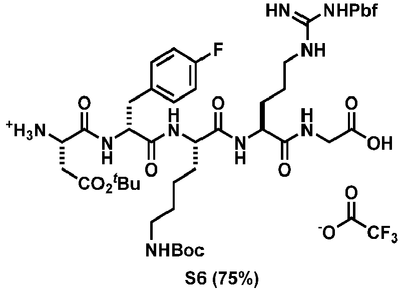

A peptide synthesis vessel (100 mL) was charged with 2-chlorotritly-chloride resin (100-200 mesh, 1% DVB, 1.6 mmol · g⁻¹, 1.00 g, 1.60 mmol, 1.00 equiv) and DCM (45 mL, 22 g · L⁻¹). The suspension was shaken with the aid of a Heidolph Vibramax 100 for 30 minutes at 23 °C. The liquid was removed via vacuum filtration, and a solution of Fmoc-Gly-OH (1.90 g, 6.40 mmol, 4.00 equiv) and DIPEA (2.79 mL, 2.07 g, 16.0 mmol, 10.0 equiv) in DCM (30 mL) was added into the peptide synthesis vessel. The resulting suspension was shaken for 15 hours at 23 °C, and then the liquid was removed via vacuum filtration. The resin was washed with DCM (3 × 20 mL × 2 min), and a solution of DIPEA, MeOH, and DCM (1:2:17, v:v:v, 30 mL) was added into the peptide synthesis vessel. The suspension was shaken for 1 hour at 23 °C, and then the resin was washed sequentially with DMF (2 × 20 mL), DCM (2 × 20 mL), MeOH (2 × 20 mL), and Et₂O (2 × 20 mL). The resin was dried under vacuum to afford Fmoc-Gly-O-2CT resin. The resin loading was determined to be 0.861 mmol · g⁻¹ by UV-vis spectroscopy.

A peptide synthesis vessel (100 mL) was charged with Fmoc-Gly-O-2CT resin (0.861 mmol · g⁻¹, 1.16 g, 1.00 mmol, 1.00 equiv) and DCM (45 mL, 26 g · L⁻¹). The resulting suspension was shaken for 30 minutes at 23 °C, and then the liquid was removed via vacuum filtration. The resin was washed with DMF (3 × 10 mL × 2 min). Into the peptide synthesis vessel was added 20% piperidine in DMF (v:v, 20 mL), and the suspension was shaken for 5 minutes at 23 °C. Then the liquid was removed via vacuum filtration. This deprotection sequence was repeated once, and then the resin was washed with DMF (3 × 20 mL × 2 min). A round-bottom flask (20 mL) equipped with a Teflon-coated magnetic stirring bar was charged with Fmoc-Asp(*^{t}*Bu)-OH (1.64 g, 4.00 mmol, 4.00 equiv), HBTU (1.48 g, 3.90 mmol, 3.90 equiv), HOBt hydrate (527 mg, 3.90 mmol, 3.90 equiv), DIPEA (1.40 mL, 1.03 g, 8.00 mmol, 8.00 equiv), and DMF (10 mL). The yellow solution was stirred for 15 minutes at 23 °C and was then added into the peptide synthesis vessel. The vessel was shaken for 90 minutes at 23 °C, and then the liquid was removed via vacuum filtration. The resin was washed with DMF (3 × 10 mL × 2 min). Into the peptide synthesis vessel was added 20% piperidine in DMF (v:v, 20 mL), and the suspension was shaken for 5 minutes at 23 °C. Then the liquid was removed via vacuum filtration. This deprotection sequence was repeated once, and then the resin was washed with DMF (3 × 20 mL × 2 min). A round-bottom flask (20 mL) equipped with a Teflon-coated magnetic stirring bar was charged with Fmoc-D-Phe(4-F)-OH (1.62 g, 4.00 mmol, 4.00 equiv), HBTU (1.48 g, 3.90 mmol, 3.90 equiv), HOBt hydrate (527 mg, 3.90 mmol, 3.90 equiv), DIPEA (1.40 mL, 1.03 g, 8.00 mmol, 8.00 equiv), and DMF (10 mL). The yellow solution was stirred for 15 minutes at 23 °C and was then added into the peptide synthesis vessel. The vessel was shaken for 90 minutes at 23 °C, and then the liquid was removed via vacuum filtration. The resin was washed with DMF (3 × 10 mL × 2 min). Into the peptide synthesis vessel was added 20% piperidine in DMF (v:v, 20 mL), and the suspension was shaken for 5 minutes at 23 °C. Then the liquid was removed via vacuum filtration. This deprotection sequence was repeated once, and then the resin was washed with DMF (3 × 20 mL × 2 min). A round-bottom flask (20 mL) equipped with a Teflon-coated magnetic stirring bar was charged with Fmoc-Lys(Boc)-OH (1.87 g, 4.00 mmol, 4.00 equiv), HBTU (1.48 g, 3.90 mmol, 3.90 equiv), HOBt hydrate (527 mg, 3.90 mmol, 3.90 equiv), DIPEA (1.40 mL, 1.03 g, 8.00 mmol, 8.00 equiv), and DMF (10 mL). The yellow solution was stirred for 15 minutes at 23 °C and was then added into the peptide synthesis vessel. The vessel was shaken for 90 minutes at 23 °C, and then the liquid was removed via vacuum filtration. The resin was washed with DMF (3 × 10 mL × 2 min). Into the peptide synthesis vessel was added 20% piperidine in DMF (v:v, 20 mL), and the suspension was shaken for 5 minutes at 23 °C. Then the liquid was removed via vacuum filtration. This deprotection sequence was repeated once, and then the resin was washed with DMF (3 × 20 mL × 2 min). A round-bottom flask (20 mL) equipped with a Teflon-coated magnetic stirring bar was charged with Fmoc-Arg(Pbf)-OH (2.60 g, 4.00 mmol, 4.00 equiv), HBTU (1.48 g, 3.90 mmol, 3.90 equiv), HOBt hydrate (527 mg, 3.90 mmol, 3.90 equiv), DIPEA (1.40 mL, 1.03 g, 8.00 mmol, 8.00 equiv), and DMF (10 mL). The yellow solution was stirred for 15 minutes at 23 °C and was then added into the peptide synthesis vessel. The vessel was shaken for 90 minutes at 23 °C, and then the liquid was removed via vacuum filtration. The resin was washed with DMF (3 × 10 mL × 2 min). Into the peptide synthesis vessel was added 20% piperidine in DMF (v:v, 20 mL), and the suspension was shaken for 5 minutes at 23 °C. Then the liquid was removed via vacuum filtration. This deprotection sequence was repeated once, and then the resin was washed with DMF (3 × 20 mL × 2 min). The resin was washed with DCM (3 × 20 mL × 2 min). Then a solution of 20% of hexafluoroisopropanol (HFIP) in DCM (v:v, 50 mL) was added to the resin, and the suspension was shaken for 20 minutes at 23 °C. The liquid was collected via vacuum filtration, and a solution of 20% of HFIP in DCM (v:v, 50 mL) was added to the resin, and the suspension was shaken for 50 minutes at 23 °C. The liquid was collected via vacuum filtration, and the combined organic layers were concentrated *in vacuo* to dryness, and were analyzed via LC-MS. The beige residue was purified by HPLC with an YMC-Actus Triart C18 column ((30 × 150 mm, 5 µm + 30 × 50 mm, 5 µm), flow rate = 42.5 mL · min⁻¹, 35 °C) with a linear gradient from 40:60 (0.1% TFA in H₂O:MeOH, v:v) to 10:90 (0.1% TFA in H₂O:MeOH, v:v) over 10 minutes. The collected fractions containing the product (t ≈ 8.5 min) were combined and concentrated *in vacuo* to dryness to afford **S6** as a colorless solid (870 mg, 0.751 mmol, 75% yield).
**HRMS-ESI (m/z)** calc'd for C₄₉H₇₃FN₉O₁₃S [M-CF₃CO₂H-H]⁻, 1046.503809; found, 1046.504640; deviation: -0.79 ppm.

### [c(Asp(^{t}Bu)-D-Phe(4-F)-Lys(Boc)-Arg(Pbf)-Gly)]·CF₃CO₂H (S7)

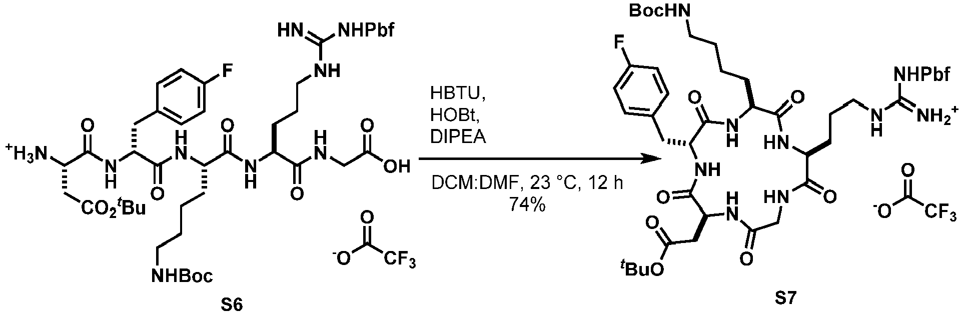

A round-bottom flask (1 L) equipped with a Teflon-coated magnetic stirring bar was charged with HOBt (119 mg, 0.878 mmol, 1.20 equiv), HBTU (332 mg, 0.878 mmol, 1.20 equiv), DIPEA (382 µL, 284 mg, 2.19 mmol, 3.00 equiv), DCM (400 mL), and DMF (100 mL). The reaction mixture was cooled to 0°C and a solution of linear peptide **S6** (850 mg, 0.731 mmol, 1.00 equiv) in DMF (20 mL) was added dropwise via a syringe over 20 minutes. The reaction mixture was allowed to warm to 23 °C, and was afterwards stirred for 12 hours at 23 °C. The solution was concentrated by rotary evaporation to 5 mL and was then diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30 × 150 mm, 5 µm + 30 × 50 mm, 5 µm), flow rate = 42.5 mL·min⁻¹, 35 °C) with a linear gradient from 30:70 (0.1% TFA in H₂O:MeOH, v:v) to 05:95 (0.1% TFA in H₂O:MeOH, v:v) over 10 minutes. The collected fractions containing the product **S7** (t ≈ 9.5 min) were combined, neutralized to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S7** a colorless powder (620 mg, 0.543 mmol, 74% yield).
**HRMS-ESI (m/z)** calc'd for C₄₉H₇₂FN₉O₁₂SNa [M+Na-CF₃CO₂H]⁺, 1052.489738; found, 1052.490630; deviation: -0.85 ppm.

### [c(Asp-D-Phe(4-F)-Lys-Arg-Gly]·2CF₃CO₂H (S8)

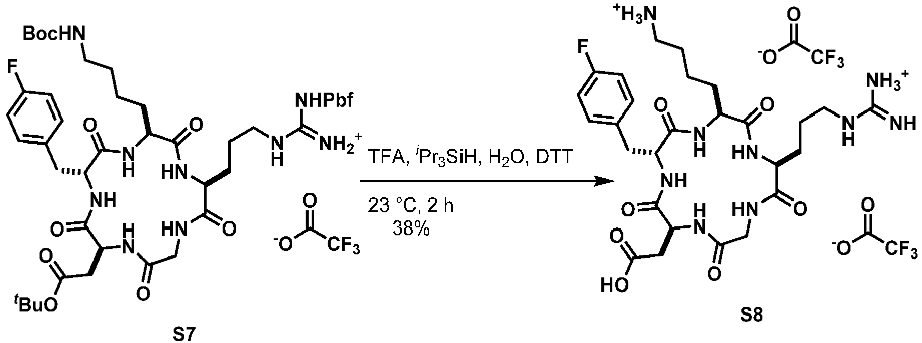

A vial (4 mL) equipped with a Teflon-coated magnetic stirring bar was charged with TFA (410 µL, 610 mg, 5.35 mmol, 102 equiv), DTT (35.0 mg, 0.227 mmol, 4.32 equiv), water (35.0 µL, 35.0 mg, 1.94 mmol, 37.0 equiv), and triisopropylsilane (17.5 µL, 13.5 mg, 85.4 µmol, 1.63 equiv). Cyclic peptide **S7** (60.0 mg, 52.5 µmol, 1.00 equiv) was added to the emulsion, and the reaction mixture was stirred at 23 °C for 2 hours. Afterwards, the reaction mixture was concentrated *in vacuo* to dryness. The residual beige solid was purified by HPLC on an YMC-Actus Triart C18 column ((30 × 150 mm, 5 µm + 30 × 50 mm, 5 µm), flow rate = 42.5 mL·min⁻¹, 35 °C) with a linear gradient from 70:30 (0.1% TFA in H₂O:MeOH, v:v) to 35:65 (0.1% TFA in H₂O:MeOH, v:v) over 10 minutes. The collected fractions containing the product (t ≈ 4.6 min) were combined and concentrated *in vacuo* to dryness to afford **S8** as a colorless solid (17.0 mg, 20.0 µmol, 38% yield).
**HRMS-ESI (m/z)** calc'd for C₂₇H₄₂FN₉O₇ [M-2·CF₃CO₂]²⁺, 311.659011; found, 311.658850; deviation: 0.52 ppm.

### [H-Asp(^{t}Bu)-D-Tyr(RuCp)-Lys(Boc)-Arg(Pbf)-Gly-OH]·CF₃CO₂H·CF₃CO₂ (S9)

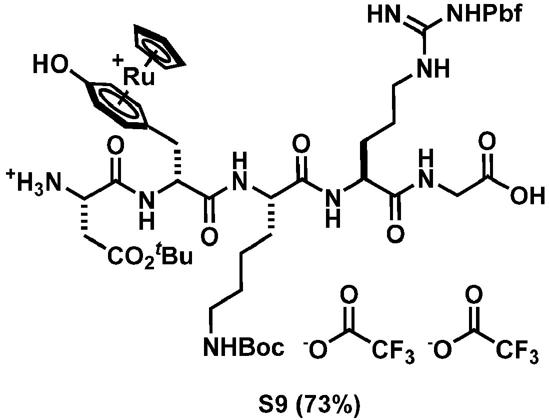

The peptide was synthesized according to the general procedure for peptide synthesis starting with 1 mmol Fmoc-Gly-O-2CT resin. The beige residue was purified by HPLC with an YMC-Actus Triart C18 column ((30×150 mm, 5 µm + 30x50 mm, 5 µm), flow rate = 42.5 mL·min⁻¹, 35 °C) with a linear gradient from 40:60 (0.1% TFA in H₂O:MeOH, v:v) to 10:90 (0.1% TFA in H₂O:MeOH, v:v) over 10 minutes. The collected fractions containing the product (t ≈ 6.5 min) were combined and concentrated *in vacuo* to dryness to afford **S9** as a colorless solid (1.05 g, 0.729 mmol, 73% yield).
**HRMS-ESI** (m/z) calc'd for C₅₄H₈₁O₁₄N₉RUS [M-2CF₃CO₂]²⁺, 606.73281; found, 606.73366; deviation: 1.40 ppm.

### [c(Asp(^{t}Bu)-D-Tyr(RuCp)-Lys(Boc)-Arg(Pbf)-Gly)] (S10)

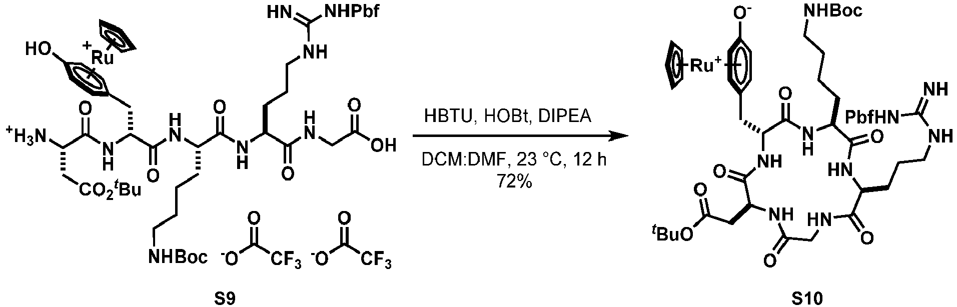

A round-bottom flask (1 L) equipped with a Teflon-coated magnetic stirring bar was charged with HOBt (104 mg, 0.767 mmol, 1.20 equiv), HBTU (291 mg, 0.767 mmol, 1.20 equiv), DIPEA (334 µL, 248 mg, 1.92 mmol, 3.00 equiv), DCM (400 mL), and DMF (100 mL). The reaction mixture was cooled to 0°C and a solution of linear peptide **S9** (920 mg, 0.639 mmol, 1.00 equiv) in DMF (20 mL) was added dropwise via a syringe over 20 min The reaction mixture was allowed to warm to 23 °C and was afterwards stirred for 12 hours at 23°C. The solution was concentrated by rotary evaporation to 5 ml and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL·min⁻¹, 35 °C) with a linear gradient from 20:80 (0.1% TFA in H₂O:MeOH, v:v) to 10:90 (0.1% TFA in H₂O:MeOH, v:v) over 10 minutes. The collected fractions containing the product (t ≈ 8.0 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S10** a colorless powder (550 mg, 0.461 mmol, 72% yield).
**HRMS-ESI** (m/z) calc'd for C₅₄H₇₈O₁₃N₉RuS [M+H]⁺, 1194.447780; found, 1194.449790; deviation: 1.68 ppm.

### [Boc-D-Glu(^{t}Bu)-Ala-Tyr(^{t}Bu)-Gly-Trp(Boc)-Met-Asp(^{t}Bu)-Phe(4-F)-OH]·CF₃CO₂H (S11)

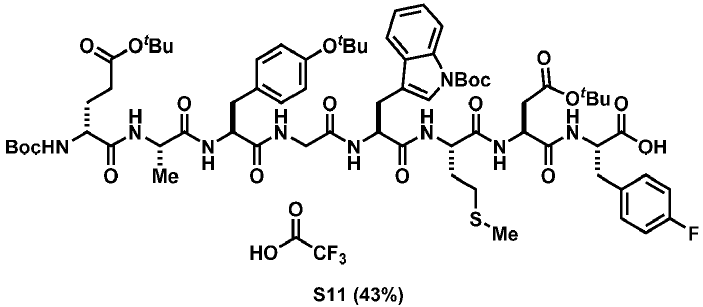

The peptide was synthesized according to the general procedure for peptide synthesis starting with 0.5 mmol Fmoc-Phe(4-F)-O-2CT resin. The beige residue was purified by HPLC with an YMC-Actus Triart C18 column ((30×150 mm, 5 µm + 30x50 mm, 5 µm), flow rate = 42.5 mL·min⁻¹, 35 °C) with a linear gradient from 15:85 (0.1% TFA in H₂O:MeOH, v:v) to 05:95 (0.1% TFA in H₂O:MeOH, v:v) over 10 minutes. The collected fractions containing the product (t ≈ 9.0 min) were combined, basified to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford the title compound **S11** as a colorless solid (323 mg, 0.213 mmol, 43% yield).
**HRMS-ESI** (m/z) calc'd for C₇₀H₉₇FN₉O₁₈S [M-CF₃CO₂H-H]⁻, 1402.666184; found, 1402.666030; deviation: 0.11 ppm.

### [Boc-D-Glu(^{t}Bu)-Ala-Tyr(^{t}Bu)-Gly-Trp(Boc)-Met-Asp(^{t}Bu)-Phe(4-F)-NHTmob]·CF₃CO₂H (S12)

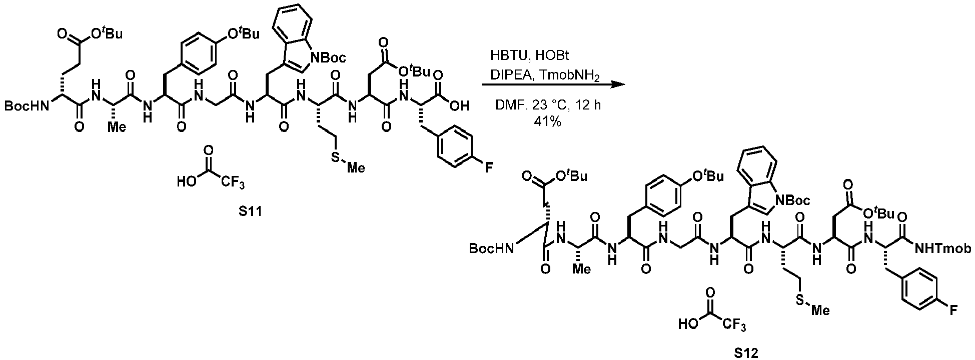

A vial (20 mL) equipped with a Teflon-coated magnetic stirring bar was charged with **S11** (264 mg, 173 µmol, 1.00 equiv), HOBt (25.8 mg, 191 µmol, 1.10 equiv), TmobNH₂ (37.7 mg, 191 µmol, 1.10 equiv), DIPEA (99.9 µL, 73.5 mg, 574 µmol, 3.30 equiv) and DMF (17 mL, 0.010 mol·L⁻¹). The reaction mixture was cooled to 0 °C and then HBTU (72.5 mg, 191 µmol, 1.10 equiv) was added. The reaction mixture was allowed to warm to room temperature and was stirred at 23 °C for 12 hours. The solution was concentrated by rotary evaporation to 5 mL, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL · min⁻¹, 35 °C) with a linear gradient from 25:75 (0.1% TFA in H₂O:MeOH, v:v) to 03:97 (0.1% TFA in H₂O:MeOH, v:v) over 10 minutes. The collected fractions containing the product (t ≈ 9.8 min) were combined, basified to pH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S12** a colorless powder (120 mg, 70.7 µmol, 41% yield).
**HRMS-ESI (m/z)** calc'd for C₈₀H₁₁₀FN₁₀O₂₀S [M-H-CF₃CO₂H]⁻, 1581.760813; found, 1581.761600.

### [H-D-Glu-Ala-Tyr-Gly-Trp-Met-Asp-Phe(4-F)-NH₂]·2CF₃CO₂H (S13)

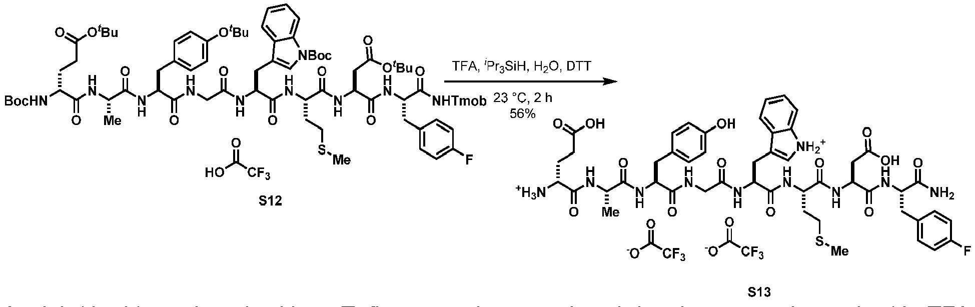

A vial (4 mL) equipped with a Teflon-coated magnetic stirring bar was charged with TFA (410 µL, 610 mg, 5.35 mmol, 152 equiv), DTT (35.0 mg, 0.227 mmol, 6.43 equiv), water (35.0 µL, 35.0 mg, 1.94 mmol, 55.1 equiv), and triisopropylsilane (17.5 µL, 13.5 mg, 85.4 µmol, 2.42 equiv). Linear peptide **S12** (59.9 mg, 35.3 µmol, 1.00 equiv) was added to the emulsion, and the reaction mixture was stirred at 23 °C for 2 hours. The reaction mixture was concentrated *in vacuo* to dryness, and the resulting beige solid was purified by HPLC on an YMC-Actus Triart C18 column ((30×150 mm, 5 µm + 30×50 mm, 5 µm), flow rate = 42.5 mL · min⁻¹, 35 °C) with a linear gradient from 50:50 (0.1% TFA in H₂O:MeOH, v:v) to 15:85 (0.1% TFA in H₂O:MeOH, v:v) over 10 minutes. The collected fractions containing **S13** (t ≈ 6.3 min) were combined and concentrated *in vacuo* to dryness to afford the **S13** as a colorless solid (25.0 mg, 19.8 µmol, 56% yield).
**HRMS-ESI (m/z)** calc'd for C₄₈H₅₈F N₁₀O₁₃S [M-H-2CF₃CO₂H]⁻, 1033.389508; found, 1033.390250; deviation: -0.72 ppm.

### [Boc-D-Glu(^{t}Bu)-Ala-Tyr(^{t}Bu)-Gly-Trp(Boc)-Met-Asp(^{t}Bu)-Tyr(RuCp)-OH]·CF₃CO₂ (S14)

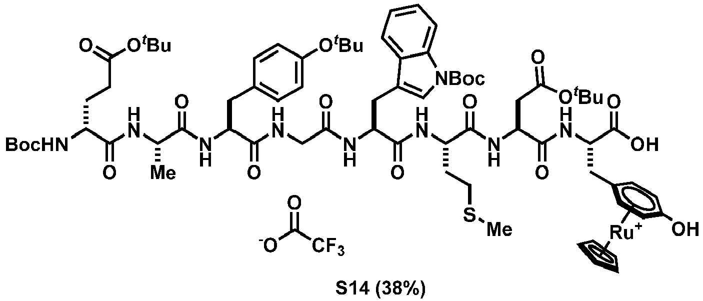

A peptide synthesis vessel (100 mL) was charged with 2-chlorotritly-chloride resin (100-200 mesh, 1% DVB, 1.6 mmol · g⁻¹, 1.00 g, 1.60 mmol, 1.00 equiv) and DCM (45 mL, 22 g · L⁻¹). The suspension was shaken with the aid of a Heidolph Vibramax 100 for 30 minutes at 23 °C. The liquid was removed via vacuum filtration, and a solution of [Fmoc-Tyr(RuCp)-O]·H₂O (1.88 g, 3.20 mmol, 2.00 equiv) and DIPEA (1.40 mL, 1.04 g, 8.00 mmol, 5.00 equiv) in DCM (30 mL) was added into the peptide synthesis vessel. The resulting suspension was
shaken for 15 hours at 23 °C, and then the liquid was removed via vacuum filtration. The resin was washed with DCM (3 × 20 mL × 2 min), and a solution of DIPEA, MeOH, and DCM (1:2:17, v:v:v, 30 mL) was added into the peptide synthesis vessel. The suspension was shaken for 1 hour at 23 °C, and then the resin was washed sequentially with DMF (2 × 20 mL), DCM (2 × 20 mL), MeOH (2 × 20 mL), and Et₂O (2 × 20 mL). The resin was dried under vacuum to afford Fmoc-Gly-O-2CT resin. The resin loading was determined to be 0.435 mmol · g⁻¹ by UV-vis spectroscopy.
The peptide was synthesized according to the general procedure for peptide synthesis starting with 1.00 mmol Fmoc-Tyr(RuCp)-O-2CT resin. The beige residue was purified by HPLC with an YMC-Actus Triart C18 column ((30×150 mm, 5 µm + 30x50 mm, 5 µm), flow rate = 42.5 mL · min⁻¹, 35 °C) with a linear gradient from 25:75 (0.1% TFA in H₂O:MeOH, v:v) to 15:85 (0.1% TFA in H₂O:MeOH, v:v) over 10 minutes. The collected fractions containing the product (t ≈ 9.5 min) were combined, neutralized to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S14** as a colorless solid (646 mg, 0.384 mmol, 38% yield).
**HRMS-ESI (m/z)** calc'd for C₇₅H₁₀₂O₁₉N₉RuS [M-H-CF₃CO₂H]⁻, 1566.60617; found, 1566.60617; deviation: -1.10 ppm.

### [Boc-D-Glu(^{t}Bu)-Ala-Tyr(^{t}Bu)-Gly-Trp(Boc)-Met-Asp(^{t}Bu)-Tyr(RuCp)-NHTmob]·CF₃CO₂ (S15)

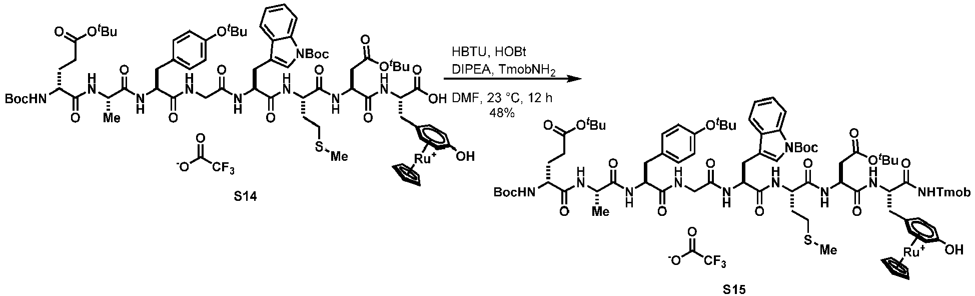

A 50 mL round-bottom flask equipped with a magnetic stirring bar was charged with **S14** (595 mg, 354 µmol, 1.00 equiv), HOBt (52.6 mg, 389 µmol, 1.10 equiv), TmobNH₂ (76.8 mg, 389 µmol, 1.10 equiv), DIPEA (203 µL, 151 mg, 1.17 mmol, 3.30 equiv) and DMF (17 mL, 0.02 mmol·L⁻¹). The reaction mixture was cooled to 0 °C and then HBTU (148 mg, 389 µmol, 1.10 equiv) was added. The reaction mixture was allowed to warm to room temperature and was stirred at 23 °C for 12 hours. The solution was concentrated by rotary evaporation to 5 ml, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL·min⁻¹, 35 °C) with a linear gradient from 20:80 (0.1% TFA in H₂O:MeOH, v:v) to 10:90 (0.1% TFA in H₂O:MeOH, v:v) over 10 minutes. The collected fractions containing the product (t ≈ 8 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S15** a colorless powder (320 mg, 172 µmol, 49% yield).
**HRMS-ESI (m/z)** calc'd for C₈₅H₁₁₅O₂₁N₁₀RuS [M-H-CF₃CO₂H]⁻, 1745.70079; found, 1745.702130; deviation: -0.77 ppm.

### [Boc-Gly-Asn(Trt)-Leu-Trp(Boc)-Ala-Thr(^{t}Bu)-Gly-His(Trt)-Phe(4-F)-Met-OH]·CF₃CO₂H (S16)

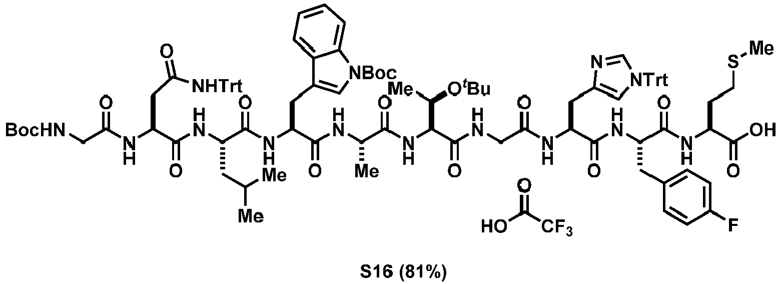

The peptide was synthesized according to the general procedure for peptide synthesis starting with 1.00 mmol Fmoc-Met-O-2CT resin. The beige residue was purified by HPLC with an YMC-Actus Triart C18 column ((30×150 mm, 5 µm + 30x50 mm, 5 µm), flow rate = 42.5 mL · min⁻¹, 35 °C) with a linear gradient from 20:80 (0.1% TFA in H₂O:MeOH, v:v) to 03:97 (0.1% TFA in H₂O:MeOH, v:v) over 6 minutes. The collected fractions containing the product (t ≈ 7.5 min) were combined, neutralized to pH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S16** as a colorless solid (1.63 g, 0.813 mmol, 81% yield).
**HRMS-ESI (m/z)** calc'd for C₁₀₄H₁₂₂FN₁₄O₁₇S [M-H-CF₃CO₂H]⁻, 1889.882263; found, 1889.881410; deviation: 0.45 ppm.

### [Boc-Gly-Asn(Trt)-Leu-Trp(Boc)-Ala-Thr(^{t}Bu)-Gly-His(Trt)-Phe(4-F)-Met-NHTmob]·2CF₃CO₂H (S17)

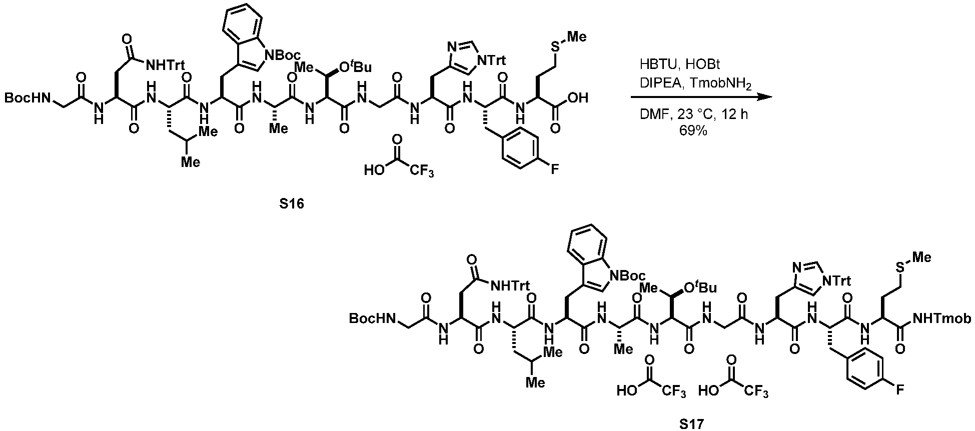

A 50 mL round-bottom flask equipped with a magnetic stirring bar was charged with **S16** (1.63 g, 863 µmol, 1.00 equiv), HOBt (128 mg, 949 µmol, 1.10 equiv), TmobNH₂ (187 mg, 949 µmol, 1.10 equiv), DIPEA (496 µL, 368 mg, 2.85 mmol, 3.30 equiv), and DMF (43 mL, 0.02 mmol · L⁻¹). The reaction mixture was cooled to 0 °C and then HBTU (360 mg, 949 µmol, 1.10 equiv) was added. The reaction mixture was allowed to warm to 23 °C and was stirred at 23 °C for 12 hours. The solution was concentrated by rotary evaporation to 5 mL and was afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL · min⁻¹, 35 °C) with a linear gradient from 20:80 (0.1% TFA in H₂O:MeOH, v:v) to 03:97 (0.1% TFA in H₂O:MeOH, v:v) over 10 minutes. The collected fractions containing the product (t ≈ 7.5 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S17** as a colorless powder (1.30 g, 0.599 mmol, 69% yield).
**HRMS-ESI (m/z)** calc'd for C₁₁₄H₁₃₅FN₁₅O₁₉S [M-H-2CF₃CO₂H]⁻, 2068.976892; found, 2068.974870; deviation: 0.98 ppm.

### [H-Gly-Asn-Leu-Trp-Ala-Thr-Gly-His-Phe(4-F)-Met-NH₂]·4CF₃CO₂H (S18)

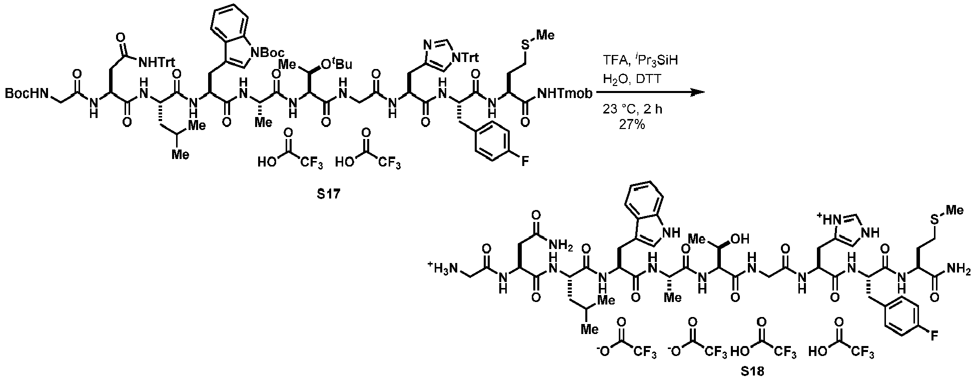

A vial (4 mL) equipped with a Teflon-coated magnetic stirring bar was charged with TFA (6.78 mL, 10.1 g, 88.5 mmol, 152 equiv), DTT (576 mg, 3.74 mmol, 6.40 equiv), water (579 µL, 579 mg, 32.1 mmol, 55.1 equiv), and triisopropylsilane (287 µL, 222 mg, 1.40 mmol, 2.40 equiv). Linear peptide **S17** (1.21 g, 584 µmol, 1.00 equiv) was added to the emulsion, and the reaction mixture was stirred at 23 °C for 2 hours. The product was precipitated by addition to rapidly stirring Et₂O (100 mL), and the off-white solid was collected by filtration. The solid was purified by HPLC on an YMC-Actus Triart C18 column ((30×150 mm, 5 µm + 30×50 mm, 5 µm), flow rate = 42.5 mL · min⁻¹, 35 °C) with a linear gradient from 50:50 (0.1% TFA in H₂O:MeOH, v:v) to 25:75 (0.1% TFA in H₂O:MeOH, v:v) over 10 minutes. The collected fractions containing the product (t ≈ 5.0 min) were combined and concentrated *in vacuo* to dryness to afford **S18** as a colorless solid (254 mg, 158 µmol, 27% yield).
**HRMS-ESI (m/z)** calc'd for C₅₂H₇₃F₁N₁₅O₁₂S₁ [M-3CF₃CO₂H-CF₃CO₂]⁺, 1150.526237; found, 1150.527210; deviation: -0.85 ppm.

### [Boc-Gly-Asn(Trt)-Leu-Trp(Boc)-Ala-Thr(^{t}Bu)-Gly-His(Trt)-Tyr(RuCp)-Met-OH]·CF₃CO₂ (S19)

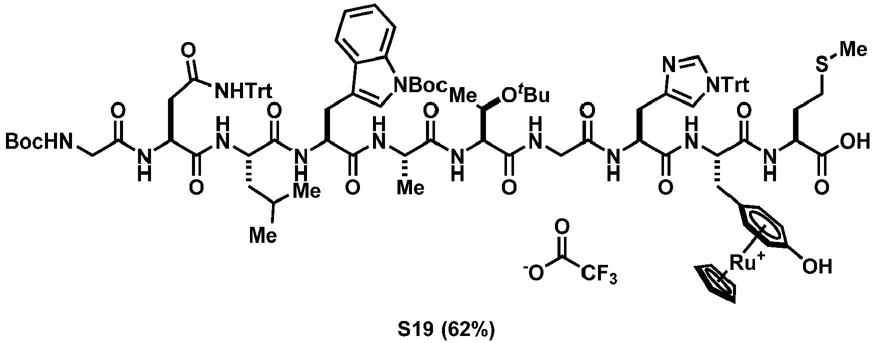

The peptide was synthesized according to the general procedure for peptide synthesis starting with 1.00 mmol Fmoc-Met-O-2CT resin. The beige residue was purified by HPLC with an YMC-Actus Triart C18 column ((30×150 mm, 5 µm + 30×50 mm, 5 µm), flow rate = 42.5 mL · min⁻¹, 35 °C) with a linear gradient from 27:63 (0.1% TFA in H₂O:MeOH, v:v) to 03:97 (0.1% TFA in H₂O:MeOH, v:v) over 10 minutes. The collected fractions containing the product (t ≈ 7.5 min) were combined, neutralized to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S19** as a colorless solid (1.35 g, 0.622 mmol, 62% yield).
**HRMS-ESI (m/z)** calc'd for C₁₀₉H₁₂₀O₁₈N₁₄SRu [M-CF₃CO₂]⁺, 2055.83680; found, 2055.840210; deviation: -1.66 ppm.

### [Boc-Gly-Asn(Trt)-Leu-Trp(Boc)-Ala-Thr(^{t}Bu)-Gly-His(Trt)-Tyr(RuCp)-Met-NHTmob]·CF₃CO₂ (S20)

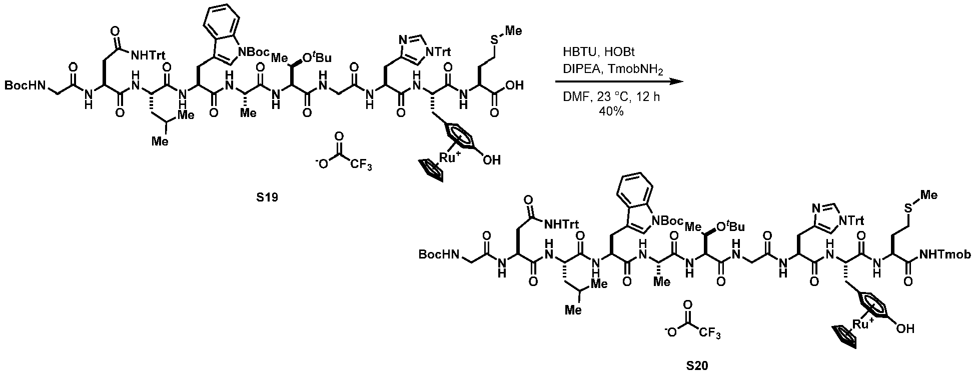

A 50 mL round-bottom flask equipped with a magnetic stirring bar was charged with **S19** (1.28 g, 591 µmol, 1.00 equiv), HOBt (87.8 mg, 0.650 mmol, 1.10 equiv), TmobNH₂ (128 mg, 0.650 mmol, 1.10 equiv), DIPEA (0.340 mL, 252 mg, 1.95 mmol, 3.30 equiv), and DMF (30 mL, 0.02 mmol · L⁻¹). The reaction mixture was cooled to 0 °C and then HBTU (246 mg, 0.650 mmol, 1.10 equiv) was added. The reaction mixture was allowed to warm to room temperature and was stirred at 23 °C for 12 hours. The solution was concentrated by rotary evaporation to 5 mL, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL · min⁻¹, 35 °C) with a linear gradient from 17:83 (0.1% TFA in H₂O:MeOH, v:v) to 14:86 (0.1% TFA in H₂O:MeOH, v:v) over 10 minutes. The collected fractions containing the product (t ≈ 9.3 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S20** as a colorless powder (558 mg, 238 µmol, 40% yield).
**HRMS-ESI (m/z)** calc'd for C₁₁₉H₁₄₂N₁₅O₂₀RuS [M-CF₃CO₂]⁺, 2234.93143; found, 2234.934870; deviation: -1.54 ppm.

### [H-Phe(4-F)-Ile-Cys-Val-Gln-Pro-Ser-Phe-OH]·CF₃CO₂H (S21)

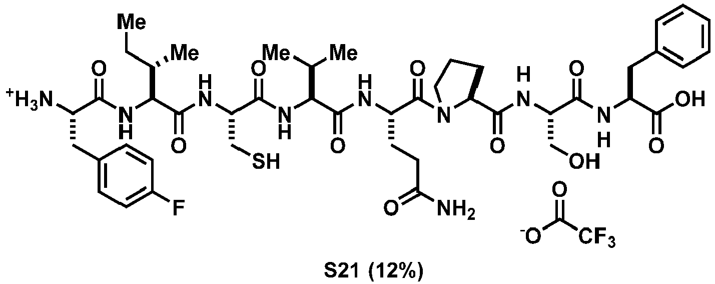

The peptide was synthesized according to the general procedure for peptide synthesis starting with 1.00 mmol Fmoc-Phe-O-2CT resin. To the beige residue was added a mixture of TFA (6.78 mL, 10.1 g, 88.5 mmol, 88.5 equiv), DTT (576 mg, 3.73 mmol, 3.73 equiv), water (579 µL, 579 mg, 32.1 mmol, 32.1 equiv), and triisopropylsilane (287 µL, 222 mg, 1.40 µmol, 1.40 equiv), and the reaction mixture was stirred at 23 °C for 2 hours. The reaction mixture was added dropwise into a round-bottom flask (100 mL) containing diethyl ether (80 mL). The resulting suspension was filtered and the filter cake was purified by HPLC on an YMC-Actus Triart C18 column ((30×150 mm, 5 µm + 30×50 mm, 5 µm), flow rate = 42.5 mL · min⁻¹, 35 °C) with a linear gradient from 27:73 (0.1% TFA in H₂O:MeOH, v:v) to 03:97 (0.1% TFA in H₂O:MeOH, v:v) over 10 minutes. The collected fractions containing the product (t ≈ 7.5 min) were combined and concentrated *in vacuo* to dryness to afford **S21** as a colorless solid (131 mg, 122 µmol, 12% yield).
**¹⁹F NMR** (471 MHz, (CD₃)₂SO, 25 °C, δ): -74.0, -116.4.
**HRMS-ESI (m/z)** calc'd for C₄₅H₆₃FN₉O₁₁S [M-H-CF₃CO₂H]⁻, 956.435730; found, 956.436390; deviation: -0.69 ppm.

### [Boc-Tyr(RuCp)-Ile-Cys(Trt)-Val-Gln(Trt)-Pro-Ser(^{t}Bu)-Phe-OH]·CF₃CO₂ (S22)

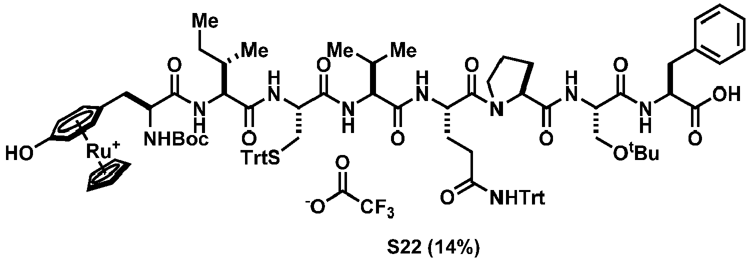

The peptide was synthesized according to the general procedure for peptide synthesis starting with 1.00 mmol Fmoc-Phe-O-2CT resin. The beige residue was purified by HPLC with an YMC-Actus Triart C18 column ((30×150 mm, 5 µm + 30×50 mm, 5 µm), flow rate = 42.5 mL·min⁻¹, 35 °C) with a linear gradient from 25:75 (0.1% TFA in H₂O:MeOH, v:v) to 15:85 (0.1% TFA in H₂O:MeOH, v:v) over 10 minutes. The collected fractions containing the product (t ≈ 6.5 min) were combined, neutralized to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S22** as a colorless solid (254 mg, 140 µmol, 14% yield).
**HRMS-ESI (m/z)** calc'd for C₉₇H₁₁₂O₁₄N₉RuS [M-H-CF₃CO₂H]⁻, 1760.709840; found, 1760.712740; deviation: -1.65 ppm.

### Boc-Leu-(4-F-Phe)-Glu(^{t}Bu)-Met-Lys(Boc)-OH (S23)

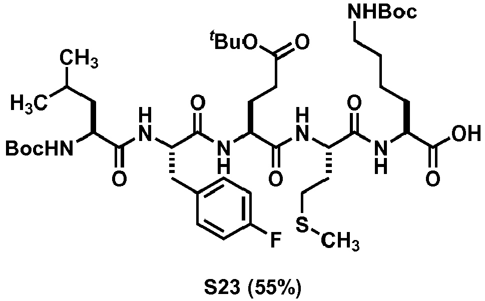

The peptide was synthesized according to the general procedure for peptide synthesis starting with 0.879 mmol Fmoc-Lys-O-2CT resin. The beige residue was purified by HPLC with an YMC-Actus Triart C18 column ((30×150 mm, 5 µm + 30×50 mm, 5 µm), flow rate = 42.5 mL · min⁻¹, 35 °C) with a linear gradient from 20:80 (0.1% TFA in H₂O:MeOH, v:v) to 03:97 (0.1% TFA in H₂O:MeOH, v:v) over 6 minutes. The collected fractions containing the product (t ≈ 7.5 min) were combined, neutralized to pH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S23** as a colorless solid (453 mg, 0.481 mmol, 55% yield).
**HRMS-ESI (m/z)** calc'd for C₄₅H₇₃O₁₂N₆FSNa [M-H-CF₃CO₂H]⁺, 963.4883; found 963.4884; deviation: -0.08 ppm.

### Boc-Leu-(4-F-Phe)-Glu(^{t}Bu)-Met-Lys(Boc)-NHTmob (S24)

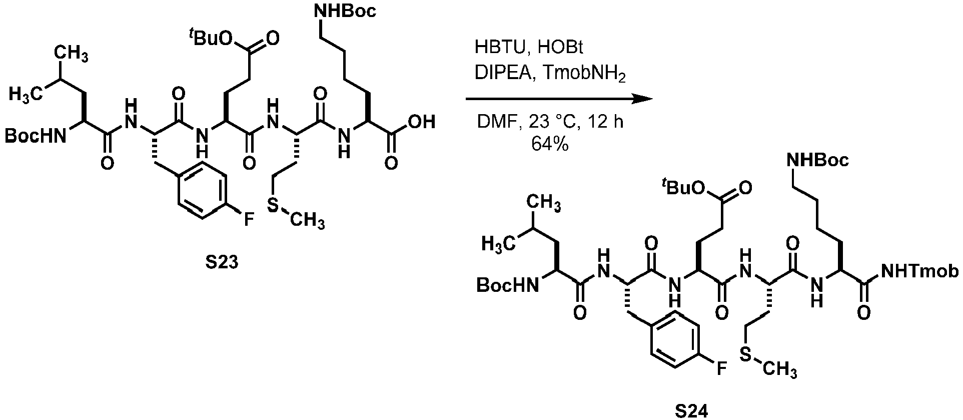

A 10 mL round-bottom flask equipped with a magnetic stirring bar was charged with **S23** (50.0 mg, 53.1 µmol, 1.00 equiv), HOBt (8.61 mg, 63.8 µmol, 1.20 equiv), TmobNH₂ (12.6 mg, 63.8 µmol, 1.20 equiv), DIPEA (33.3 µL, 24.7 mg, 191 µmol, 3.30 equiv), and DMF (2.7 mL, 20 mmol · L⁻¹). The reaction mixture was cooled to 0 °C and then HBTU (24.2 mg, 63.8 µmol, 1.20 equiv) was added. The reaction mixture was allowed to warm to room temperature and was stirred at 23 °C for 12 hours. The solution was diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC-Actus Triart C18 column ((30×150 mm, 5 µm + 30×50 mm, 5 µm), flow rate = 42.5 mL · min⁻¹, 35 °C) with a linear gradient from 17:83 (0.1% TFA in H₂O:MeOH, v:v) to 14:86 (0.1% TFA in H₂O:MeOH, v:v) over 10 minutes. The collected fractions containing the product (t ≈ 9.3 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S24** as a colorless powder (38.0 mg, 33.9 µmol, 64% yield).
**HRMS-ESI (m/z)** calc'd for C₅₅H₈₆O₁₄N₇FSNa [M-H-CF₃CO₂H]⁺, 1142.5830; 1142.5839; deviation: -0.78 ppm.

### [H-Leu-Phe(4-F)-Glu-Met-Lys-NH₂]·3CF₃CO₂H (S25)

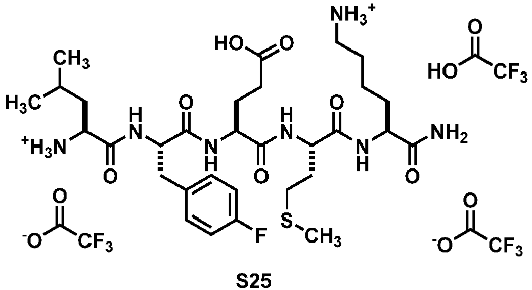

The peptide was synthesized according to the general procedure for peptide synthesis on Fmoc-Rink-Amid-2CT resin (200-400 mesh, 1% DVB, 0.68 mmol·g⁻¹, 368 mg, 250 µmol, 1.00 equiv). Different to the regular cleavage process a mixture of TFA (8.80 mL, 13.1 g, 115 mmol, 460 equiv), DTT (500 mg, 3.24 mmol, 13.0 equiv), water (500 µL, 500 mg, 27.8 mmol, 111 equiv), and triisopropylsilane (250 µL, 193 mg, 1.22 mmol, 4.88 equiv) was added, and the suspension was shacked at 23 °C for 2 hours. The reaction mixture was filtered into a round-bottom flask (100 mL) containing diethyl ether (80 mL). The resulting suspension was filtered and the filter cake was purified by HPLC on an YMC-Actus Triart C18 column ((30×150 mm, 5 µm + 30×50 mm, 5 µm), flow rate = 42.5 mL·min⁻¹, 35 °C) with a linear gradient from 50:50 (0.1% TFA in H₂O:MeOH, v:v) to 03:97 (0.1% TFA in H₂O:MeOH, v:v) over 10 minutes. The collected fractions containing the product (t ≈ 5.0 min) were combined and concentrated *in vacuo* to dryness to afford **S25** as a colorless solid (114 mg, 111 µmol, 45% yield).
**HRMS-ESI (m/z)** calc'd for C₃₁H₅₁FN₇O₇S [M-H-3CF₃CO₂H]⁻, 684.354922; found, 684.355080; deviation: -0.23 ppm.

### [Boc-Leu-Tyr(RuCp)-Glu(^{t}Bu)-Met-Lys(Boc)-OH]·CF₃CO₂ (S26)

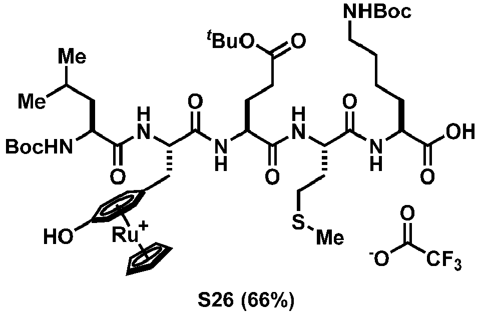

The peptide was synthesized according to the general procedure for peptide synthesis starting with 1.00 mmol Fmoc-Lys(Boc)-O-2CT resin. The beige residue was purified by HPLC with an YMC-Actus Triart C18 column ((30×150 mm, 5 µm + 30x50 mm, 5 µm), flow rate = 42.5 mL·min⁻¹, 35 °C) with a linear gradient from 30:70 (0.1% TFA in H₂O:MeOH, v:v) to 10:90 (0.1% TFA in H₂O:MeOH, v:v) over 10 minutes. The collected fractions containing the product (t ≈ 6.5 min) were combined, neutralized to pH 7 with saturated aqueous sodium bicarbonate solution, diluted with 100 mL brine, and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S26** as a colorless solid (804 mg, 0.660 mmol, 66% yield).
**HRMS-ESI (m/z)** calc'd for C₅₀H₇₉O₁₃N₆RuS [M-CF₃CO₂]⁺, 1105.44638; found, 1105.448110; deviation: -1.56 ppm.

### Boc-Leu-Tyr(RuCp)-Glu(^{t}Bu)-Met-Lys(Boc)-NHTmob (5)

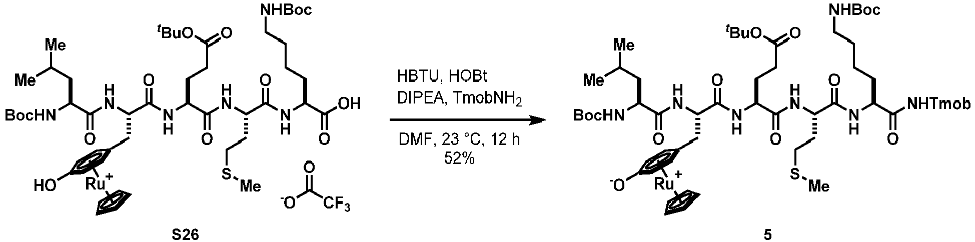

A 50 mL round-bottom flask equipped with a magnetic stirring bar was charged with **S26** (804 mg, 656 µmol, 1.00 equiv), HOBt (103 mg, 759 µmol, 1.15 equiv), TmobNH₂ (156 mg, 792 µmol, 1.20 equiv), DIPEA (414 µL, 307 mg, 2.38 mmol, 3.60 equiv), and DMF (33 mL, 20 µmol·L⁻¹). The reaction mixture was cooled to 0 °C and then HBTU (288 mg, 759 µmol, 1.15 equiv) was added. The reaction mixture was allowed to warm to room temperature and was stirred at 23 °C for 12 hours. The solution was concentrated by rotary evaporation to 5 mL, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC on an YMC Pro C18 column ((30×150 mm, 5 µm + 30x50 mm, 5 µm), flow rate = 42.5 mL · min⁻¹, 35 °C) with an isocratic eluent 30:70 (0.1% TFA in H₂O:MeOH, v:v). The collected fractions containing the product (t ≈ 14.5 min) were combined, basified to pH 8 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **5** as a colorless powder (483 mg, 346 µmol, 52% yield).
**HRMS-ESI (m/z)** calc'd for C₆₀H₉₂N₇O₁₅RuS [M-H-CF₃CO₂H]⁺, 1284.54101; found, 1284.541360; deviation: -0.27 ppm.

### Boc-Gly-His(Trt)-Gly-D-Phe(4-F)-Gly-NHTmob (S27)

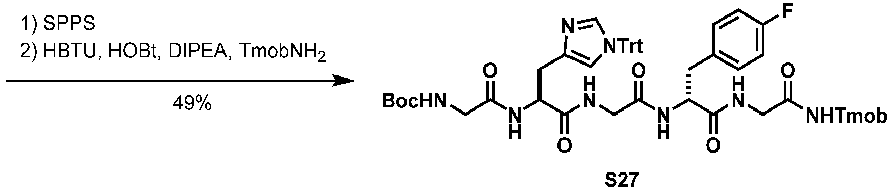

The peptide was synthesized according to the general procedure for peptide synthesis starting with 0.25 mmol Fmoc-Gly-O-2CT resin. A 50 mL round-bottom flask equipped with a magnetic stirring bar was charged with the solid residue from SPPS, HOBt (40.5 mg, 0.300 mmol, 1.20 equiv), TmobNH₂ (54.2 mg, 275 µmol, 1.10 equiv), DIPEA (131 µL, 96.9 mg, 0.750 mmol, 3.00 equiv), and DMF (12.5 mL, 0.02 mmol·L⁻¹). The reaction mixture was cooled to 0 °C and then HBTU (114 mg, 0.300 mmol, 1.20 equiv) was added. The reaction mixture was allowed to warm to room temperature and was stirred at 23 °C for 12 hours. The solution was concentrated by rotary evaporation to 5 ml, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beige residue was purified by HPLC with an YMC Pro C18 column ((30×150 mm, 5 µm + 30x50 mm, 5 µm), flow rate = 42.5 mL · min⁻¹, 35 °C) with a linear gradient from 40:60 (0.1% TFA in H₂O:MeOH, v:v) to 20:80 (0.1% TFA in H₂O:MeOH, v:v) over 10 minutes. The collected fractions containing the product (t ≈ 8.0 min) were combined, neutralized to pH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S27** as a colorless solid (123 mg, 0.121 mmol, 49% yield).
**HRMS-ESI (m/z)** calc'd for C₅₅H₆₂FN₈O₁₀ [M+H]⁺, 1013.456743; found, 1013.457120; deviation: -0.37 ppm.

### [H-Gly-His-Gly-D-Phe(4-F)-Gly-NH₂]·2CF₃CO₂H (S28)

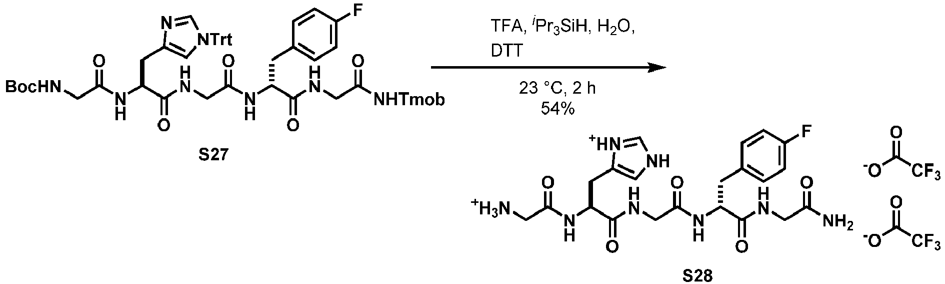

A vial (4 mL) equipped with a Teflon-coated magnetic stirring bar was charged with TFA (4.40 mL, 6.55 g, 57.5 mmol, 582 equiv), DTT (0.250 g, 1.62 mmol, 16.4 equiv), water (0.250 mL, 0.250 g, 13.9 mmol, 140 equiv), and triisopropylsilane (125 µL, 96.6 mg, 0.610 mmol, 6.18 equiv). Peptide **S27** (0.100 g, 98.7 µmol, 1.00 equiv) was added to the emulsion, and the reaction mixture was stirred at 23 °C for 2 hours. The reaction mixture was concentrated *in vacuo* and the solid residue was purified by HPLC on an Chiralpak® ZWIX (+) ((10×250 mm, 5 µm), flow rate = 6.0 mL · min⁻¹, 35 °C) with an isocratic eluent 49:49:2 (MeOH:MeCN:H₂O+50 mM formic acid, 25 mM diethylamine). The collected fractions containing the product (t ≈ 15.0 min) were combined and concentrated *in vacuo* to dryness to afford **S28** as a colorless solid (38.0 mg, 52.9 µmol, 54% yield).
**HRMS-ESI (m/z)** calc'd for C₂₁H₂₈FN₈O₅ [M-CF₃CO₂-CF₃CO₂H]⁺, 491.216118; found, 491.216180; deviation: -0.13 ppm.

### Boc-Gly-His(Trt)-Gly-Phe(4-F)-Gly-NHTmob (S29)

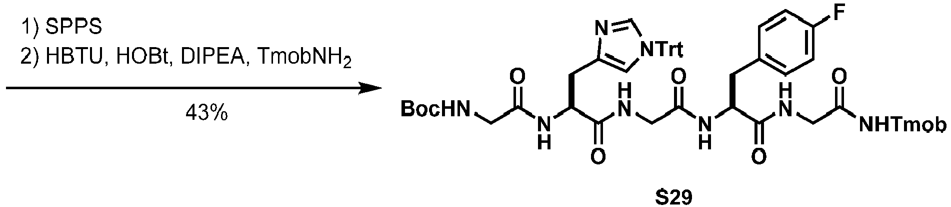

The peptide was synthesized according to the general procedure for peptide synthesis starting with 0.25 mmol Fmoc-Gly-O-2CT resin. A 50 mL round-bottom flask equipped with a magnetic stirring bar was charged solid residue from SPPS, HOBt (40.5 mg, 300 µmol, 1.20 equiv), TmobNH₂ (54.2 mg, 275 µmol, 1.10 equiv), DIPEA (414 µL, 307 mg, 2.38 mmol, 3.60 equiv), and DMF (12.5 mL, 0.2 mmol·L⁻¹). The reaction mixture was cooled to 0 °C and then HBTU (114 mg, 300 µmol, 1.20 equiv) was added. The reaction mixture was allowed to warm to room temperature and was stirred at 23 °C for 12 hours. The solution was concentrated by rotary evaporation to 5 ml, and afterwards diluted with ethyl acetate (50 mL). The solution was extracted with water (2 × 30 mL), and the combined aqueous layers were extracted with ethyl acetate (10 mL). The combined organic layers were concentrated to dryness by rotary evaporation. The beigeish residue was purified by HPLC with an YMC Pro C18 column ((30×150 mm, 5 µm + 30×50 mm, 5 µm), flow rate = 42.5 mL·min⁻¹, 35 °C) with a linear gradient from 40:60 (0.1% TFA in H₂O:MeOH, v:v) to 20:80 (0.1% TFA in H₂O:MeOH, v:v) over 10 minutes. The collected fractions containing the product (t ≈ 8.0 min) were combined, neutralized to pH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S29** as a colorless solid (110 mg, 0.660 mmol, 43% yield). **HRMS-ESI (m/z)** calc'd for C₅₅H₆₂FN₈O₁₀ [M+H]⁺, 1013.456743; found, 1013.457740; deviation: -0.98 ppm.

### [H-Gly-His-Gly-Phe(4-F)-Gly-NH₂]·2CF₃CO₂H (S30)

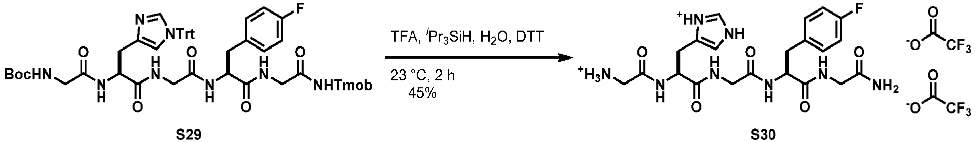

A vial (4 mL) equipped with a Teflon-coated magnetic stirring bar was charged with TFA (4.40 mL, 6.55 g, 57.5 mmol, 582 equiv), DTT (250 mg, 1.62 mmol, 16.4 equiv), water (250 µL, 250 mg, 13.9 mmol, 140 equiv), and triisopropylsilane (125 µL, 96.6 mg, 610 µmol, 6.18 equiv). Peptide **S29** (100 mg, 98.7 µmol, 1.00 equiv) was added to the emulsion, and the reaction mixture was stirred at 23 °C for 2 hours. The reaction mixture was concentrated *in vacuo* and the solid residue was purified by HPLC on an Chiralpak® ZWIX (+) ((10×250 mm, 5 µm), flow rate = 6.0 mL·min⁻¹, 35 °C) with an isocratic eluent 49:49:2 (MeOH:MeCN:H₂O+50 mM formic acid, 25 mM diethylamine). The collected fractions containing the product (t ≈ 17.5 min) were combined and concentrated *in vacuo* to dryness to afford **S30** as a colorless solid (32.0 mg, 44.5 µmol, 45% yield).
**HRMS-ESI (m/z)** calc'd for C₂₁H₂₈FN₈O₅ [M-CF₃CO₂]⁺, 491.216118; found, 491.216050; deviation: 0.14 ppm.

### [Boc-Gly-His(Trt)-Gly-Tyr(RuCp)-Gly-OH]·CF₃CO₂ (S31)

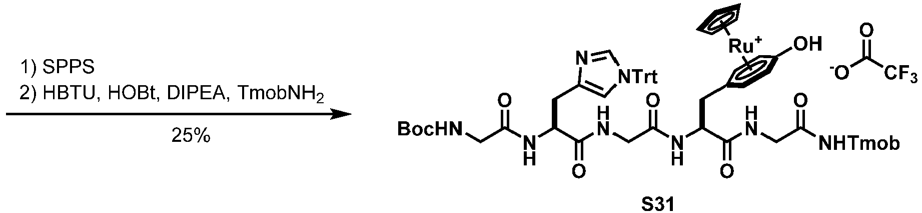

The peptide was synthesized according to the general procedure for peptide synthesis starting with 0.50 mmol Fmoc-Gly-O-2CT resin. The beige residue was purified by HPLC with an YMC Pro C18 column ((30×150 mm, 5 µm + 30x50 mm, 5 µm), flow rate = 42.5 mL·min⁻¹, 35 °C) with a linear gradient from 30:70 (0.1% TFA in H₂O:MeOH, v:v) to 05:95 (0.1% TFA in H₂O:MeOH, v:v) over 10 minutes. The collected fractions containing the product (t ≈ 4.5 min) were combined, neutralized to PH 7 with saturated aqueous sodium bicarbonate solution, diluted with 100 mL brine and the resulting solution was concentrated by rotary evaporation (100 mbar, 35 °C) until no more methanol was evaporated. The suspension was extracted with dichloromethane (3 × 100 mL), and the combined organic layers were dried over sodium sulfate, filtered, and concentrated *in vacuo* to dryness to afford **S31** as a colorless solid (158 mg, 0.122 mmol, 25% yield).
**HRMS-ESI (m/z)** calc'd for C₆₀H₆₇O₁₁N₈Ru [M-CF₃CO₂]⁺, 1177.39776; found, 1177.3978; deviation: -0.87 ppm.

### Radiochemistry general methods

No-carrier-added [¹⁸F]fluoride was purchased from Advanced Accelerator Applications SA. Liquid chromatographic analysis (LC) was performed with Thermo Scientific Dionex UltiMate 3000 dual channel HPLC system connected to LabLogic Nal/PMT-radiodetectors with Flow-Ram output. A Thermo Scientific Accucore XL C18, 4 µm, 3×150 mm HPLC column was used for analytical analysis and a Thermo Scientifc Hypersil Gold column, 5 µm, 10×250 mm HPLC column was used for preparative HPLC. Analytical and preparative HPLC used the following mobile phases: 0.1% CF₃CO₂H in water (A), 0.1% CF₃CO₂H in acetonitrile (B).

| **Gradient 1** | | | | **Gradient 2** | | |
|---|---|---|---|---|---|---|
| Time [min] | A [%] | B [%] | | Time [min] | A [%] | B [%] |
| 0 | 95 | 5 | | 0 | 95 | 5 |
| 2 | 95 | 5 | | 2 | 95 | 5 |
| 22 | 50 | 50 | | 10 | 5 | 95 |
| 22.5 | 5 | 95 | | 14 | 5 | 95 |
| 27 | 5 | 95 | | 15 | 95 | 5 |
| 28 | 95 | 5 | | 18 | 95 | 5 |
| 32 | 95 | 5 | | | | |

All ¹⁸F-labeled molecules were characterized by comparing the HPLC radio-trace of the isolated compound to the HPLC UV-trace of an authentic reference sample. Radioactivity was measured in a Veenstra Instruments, VIK-203 ionization-chamber.
Note: radioactivity chromatographs are offset by 0.1 and 0.3 minutes on account of the delay introduced by the spatial separation between the diode array detectors and the radioactivity detectors.

### General procedure for pre-conditioning of Sep-Pak cartridges

Waters Sep-Pak light C18 cartridge (part # WAT023501) was pre-conditioned by sequentially pushing MeOH (2 mL) and water (10 mL) through the cartridge unless otherwise indicated.

### General procedure for pre-conditioning of QMA cartridges

Chromafix PS-HCO₃ ¹⁸F separation cartridge (45 mg) (Product No. 731876 from ABX) was pre-conditioned by sequentially pushing potassium oxalate solution (3 mL, 10 mg · mL⁻¹ H₂O) and H₂O (2 mL) through the cartridge at a flow rate of 5 mL·min⁻¹ unless otherwise indicated.

### General procedure for radio-deoxyfluorination of peptides

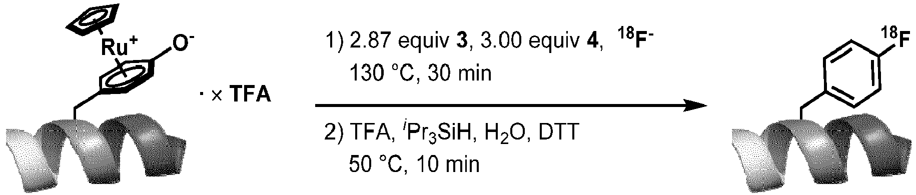

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of tracer precursor (5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (∼5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with the HPLC eluent and purified by HPLC on a Hypersil Gold column (250×10 mm, 5 µm). The activity of the product containing fraction was measured and the product identity and purity was determined by comparison of the HPLC radio-trace with the HPLC UV-trace of the authentic reference sample.

### c(Asp-D-Phe(4-[¹⁸F]F)-Lys-Arg-Gly) (7)

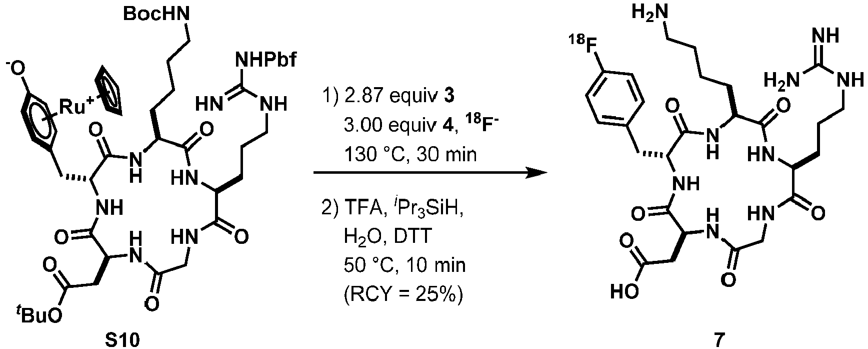

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **S10** (5.97 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250x10 mm, 5 µm, flow rate = 4 mL·min⁻¹) with an isocratic mixture of 05:95:0.1 (MeCN:water:TFA, v:v:v) for 5 minutes, followed by a linear gradient to 35:65:0.1 (MeCN:water:TFA, v:v:v) within 18 minutes. The activity of the product **7** containing fraction (≈ 22 min) was measured and the product identity and purity was determined by comparison of the HPLC radio-trace with the HPLC UV-trace of the authentic reference sample **S8**.

**Table S1. Radiochemical yield of 7**

| **Reaction** | **Starting Activity / (MBq)** | **Isolated Acitivty after HPLC / (MBq)** | **Synthesis Time / (min)** | **RCY** |
|---|---|---|---|---|
| **1** | 60.1 | 7.5 | 146 | 31% |
| **2** | 64.8 | 6.66 | 100 | 19% |
| **Average RCY** | | | | 25% |

### H-Phe(4-[¹⁸F]F)-Ile-Cys-Val-Gln-Pro-Ser-Phe-OH (8)

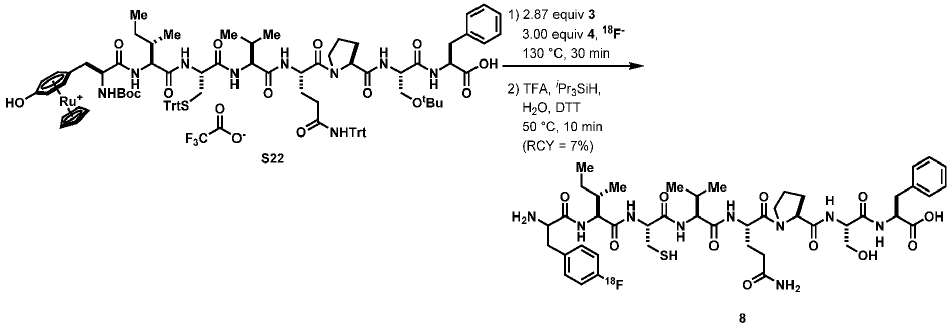

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **S22** (8.81 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250×10 mm, 5 µm, flow rate = 4 mL·min⁻¹) with an isocratic mixture of 25:75:0.1 (MeCN:water:TFA, v:v:v) for 2 minutes, followed by a linear gradient to 50:50:0.1 (MeCN:water:TFA, v:v:v) within 20 minutes. The activity of the product **8** containing fraction (≈ 21 min) was measured and the product identity and purity was determined by comparison of the HPLC radio-trace with the HPLC UV-trace of the authentic reference sample **S21**.

**Table S2. Radiochemical yield of 8**

| **Reaction** | **Starting Activity / (MBq)** | **Isolated Acitivty after HPLC / (MBq)** | **Synthesis Time / (min)** | **RCY** |
|---|---|---|---|---|
| **1** | 63.1 | 2.56 | 115 | 8% |
| **2** | 61.9 | 1.98 | 94 | 6% |
| **Average RCY** | | | | 7% |

### H-D-Glu-Ala-Tyr-Gly-Trp-Met-Asp-Phe(4-[¹⁸F]F)-NH₂ (9)

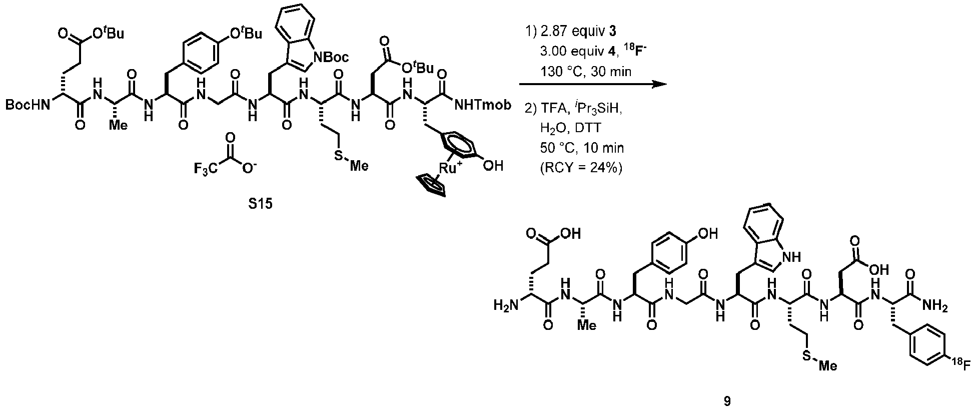

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **S15** (9.31 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250x10 mm, 5 µm, flow rate = 4 mL·min⁻¹) with an isocratic mixture of 20:80:0.1 (MeCN:water:TFA, v:v:v) for 5 minutes, followed by a linear gradient to 50:50:0.1 (MeCN:water:TFA, v:v:v) within 20 minutes. The activity of the product **9** containing fraction (≈ 24 min) was measured and the product identity and purity was determined by comparison of the HPLC radio-trace with the HPLC UV-trace of the authentic reference sample **S13**.

**Table S3. Radiochemical yield of 9**

| **Reaction** | **Starting Activity / (MBq)** | **Isolated Acitivty after HPLC / (MBq)** | **Synthesis Time / (min)** | **RCY** |
|---|---|---|---|---|
| **1** | 58.8 | 5.7 | 168 | 28% |
| **2** | 57.2 | 7.01 | 114 | 24% |
| **3** | 59.4 | 4.95 | 148 | 21% |
| **Average RCY** | | | | 24% |

### H-Gly-Asn-Leu-Trp-Ala-Thr-Gly-His-Phe(4-[¹⁸F]F)-Met-NH₂ (10)

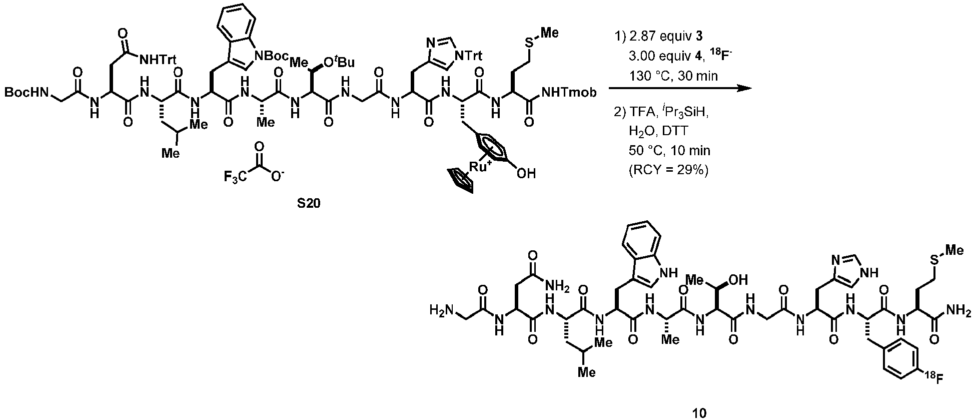

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **S20** (11.7 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250x10 mm, 5 µm, flow rate = 4 mL·min⁻¹) with an isocratic mixture of 10:90:0.1 (MeCN:water:TFA, v:v:v) for 2 minutes, followed by a linear gradient to 50:50:0.1 (MeCN:water:TFA, v:v:v) within 18 minutes. The activity of the product **10** containing fraction (≈ 23 min) was measured and the product identity and purity was determined by comparison of the HPLC radio-trace with the HPLC UV-trace of the authentic reference sample **S18.**

**Table S4. Radiochemical yield of 10**

| **Reaction** | **Starting Activity / (MBq)** | **Isolated Acitivty after HPLC / (MBq)** | **Synthesis Time / (min)** | **RCY** |
|---|---|---|---|---|
| **1** | 56.8 | 5.16 | 163 | 25% |
| **2** | 68.9 | 11.4 | 119 | 33% |
| **Average RCY** | | | | 29% |

### [¹⁸F]-H-Gly-His-Gly-Phe(4-F)-Gly-NH₂ (11)

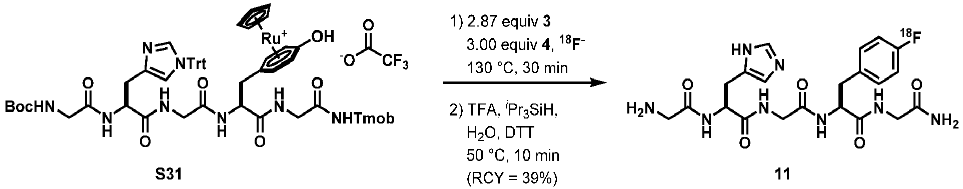

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **S31** (5.97 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250x10 mm, 5 µm, flow rate = 4 mL·min⁻¹) with an isocratic mixture of 05:95:0.1 (MeCN:water:TFA, v:v:v) for 2 minutes, followed by a linear gradient to 50:50:0.1 (MeCN:water:TFA, v:v:v) within 20 minutes. The activity of the product **11** containing fraction (≈ 19 min) was measured and the product identity and purity was determined by comparison of the HPLC radio-trace with the HPLC UV-trace of the authentic reference sample **S30.**

**Table S5. Radiochemical yield of 11**

| **Reaction** | **Starting Activity / (MBq)** | **Isolated Acitivty after HPLC / (MBq)** | **Synthesis Time / (min)** | **RCY** |
|---|---|---|---|---|
| **1** | 13.3 | 3.17 | 84 | 41% |
| **2** | 46.6 | 8.84 | 110 | 37% |
| **Average RCY** | | | | 39% |

### H-Leu-Phe(4-[¹⁸F]F)-Glu-Met-Lys-NH₂ (12)

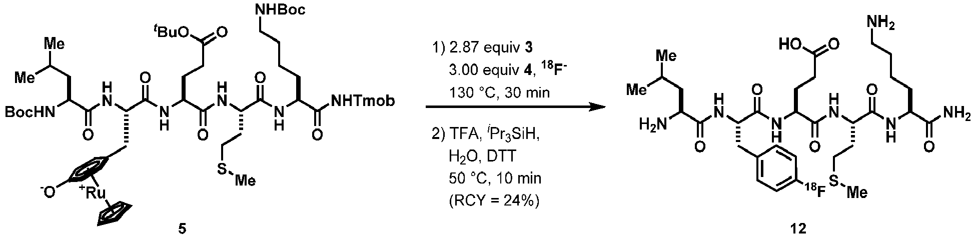

Aqueous ¹⁸F-fluoride solution (700 µL) was loaded with a syringe onto a QMA anion-exchange cartridge that is pre-conditioned according to the general procedure, and then the cartridge was washed with MeCN (1.0 mL). The ¹⁸F-fluoride was eluted from the cartridge with a solution of **5** (6.99 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and pivalonitrile (200 µL, 1:3, v:v), into a 4 mL borosilicate vial. The remaining substrate was eluted from the cartridge with a mixture of veratrole and pivalonitrile (250 µL, 4:1, v:v) into the same vial. The reaction vial containing 450 µL reaction mixture was sealed with a Teflon-lined cap and was stirred at 130 °C for 30 minutes. The vial was removed from the hot plate, and after 2 minutes the reaction mixture was concentrated by heating at 80 °C under a stream of nitrogen (5 min). To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with water (2 mL) and purified by HPLC on a Hypersil Gold column (250x10 mm, 5 µm, flow rate = 4 mL·min⁻¹) with an isocratic mixture of 15:85:0.1 (MeCN:water:TFA, v:v:v) for 2 minutes, followed by a linear gradient to 35:65:0.1 (MeCN:water:TFA, v:v:v) within 16 minutes. The activity of the product **12** containing fraction (≈ 18 min) was measured and the product identity and purity was determined by comparison of the HPLC radio-trace with the HPLC UV-trace of the authentic reference sample **S22.**

**Table S6. Radiochemical yield of 12**

| **Reaction** | **Starting Activity / (MBq)** | **Isolated Acitivty after HPLC / (MBq)** | **Synthesis Time / (min)** | **RCY** |
|---|---|---|---|---|
| **1** | 56.1 | 4.54 | 110 | 16% |
| **2** | 63.1 | 8.16 | 98 | 24% |
| **3** | 1515 | 193 | 164 | 36% |
| **Average RCY** | | | | 24% |

### Automated H-Leu-Phe(4-[¹⁸F]F)-Glu-Met-Lys-NH₂ (12) synthesis

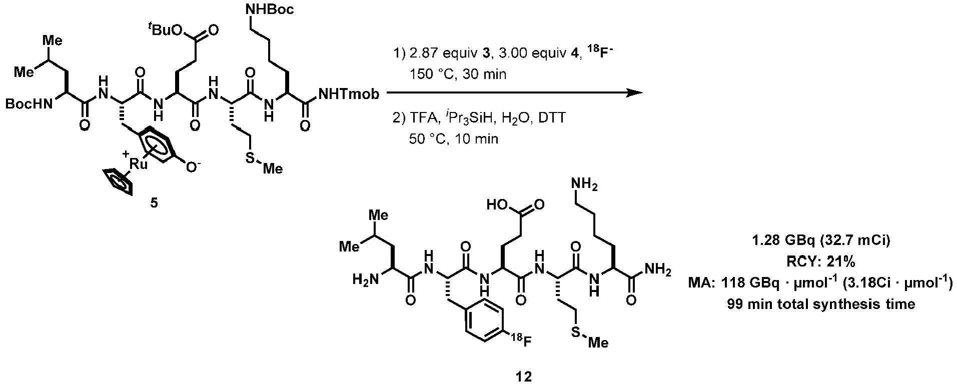

On an automated cassette-based radiochemical synthesizer Elixys FLEX/CHEM connected to a PURE/FORM purification and formulation unit (Sofie Biosciences), aqueous ¹⁸F-fluoride (11.4 GBq, (308 mCi), t = 0) was trapped onto a QMA anion-exchange cartridge that is pre-conditioned according to the general procedure, and then the cartridge was washed with MeCN (1.0 mL). The ¹⁸F-fluoride was eluted from the cartridge with a solution of **5** (5.97 mg, 5.00 µmol, 1.00 equiv), imidazolium chloride **4** (6.89 mg, 15.0 µmol, 3.00 equiv), and bis(neopentyltrimethylammonium) oxalate **3** (5.00 mg, 14.3 µmol, 2.87 equiv) in a mixture of ethanol and but-2-one (200 µL, 1:3, v:v), into a 4 mL borosilicate vial. The remaining substrate was eluted from the cartridge with a mixture of veratrole and but-2-one (250 µL, 4:1, v:v) into the same vial. The reaction vial containing 450 µL reaction mixture was sealed against a Teflon-liner and was stirred at elevated temperature (set-point at 150 °C) for 30 minutes and subsequently cooled to 50 °C. The solvent was evaporated at 100 °C under reduced pressure for 5 minutes To the remaining solution was added a mixture of TFA (420 µL), triisopropylsilane (40.0 µL), *DL*-1,4-dithiothreitol (35.0 mg), and water (20.0 µL), then the mixture was stirred at 50 °C for 10 minutes The reaction mixture was diluted with a solution of water (1.5 mL) and methanol (750µL). The reaction mixture was transferred from the Elyxsi FLEX/CHEM system to the PURE/FORM, and purified by HPLC with a Hypersil Gold (250x10 mm, 5 µm, flow rate = 4 mL·min⁻¹) column with an isocratic mixture of 15:85:0.1 (MeCN:water:TFA, v:v:v) for 2 minutes, followed by a linear gradient to 45:55:0.1 (MeCN:water:TFA, v:v:v) within 18 minutes. The activity of the product containing fraction (≈ 22 min) was diluted with water (40 mL) and loaded onto a C-18 light SepPak cartridge. The cartridge was washed with water (3 mL) and the product eluted with ethanol (1.6 mL). The ethanol solution contained 1.28 GBq (32.7 mCi, t = 99 min). The total decay corrected radio chemical yield is 21 %.The radiochemical identity was confirmed by analytical HPLC.

To determine the molar activity of **12**, 22.0 MBq of purified compound **12** was injected into an analytical HPLC and the UV absorption (2.1591 mAU×min) corresponding to the radio-peak was measured. To determine the amount of material a calibration curve was acquired with an authentic standard for H-Leu-Phe(4-F)-Glu-Met-Lys-NH₂ **S22**. It was calculated that the absorption of (2.1591 mAU×min) corresponds to 0.164 nmol of **12** and therefore the molar activity of **12** was determined to be 118 GBq · µmol⁻¹ (3.18 Ci · µmol⁻¹).
1. A [P-AR¹(RuCp)-OH] complex having the general formula (I): 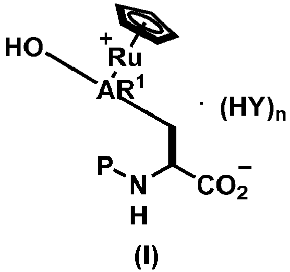
   wherein AR¹ is an aromatic or heteroaromatic hydrocarbon having 5 to 14 carbon atoms, preferably six to ten carbon atoms, which may be further substituted by at least one C₁ to C₆ alkyl group and/or by at least one heteroatom,
   wherein Y is an anion, preferably selected from CF₃CO₂⁻, triflate⁻ or OH⁻, n is 0 or 1, and P is a protective group, preferably a fluorenyl methoxy carbonyl (Fmoc), *tert*-butoxycarbonyl (Boc), or benzyloxycarbonyl (Cbz) group.
2. A [P-AR¹(RuCp)-OH] complex having the general formula (I) as defined in claim 1, wherein AR¹ is phenyl, 4-hydroxy-phenyl- or 1*H*-indol-3-yl, and Y, n and P have the meaning as defined in claim 1.
3. A [AR¹(RuCp)-OH] complex having the general formula (IV): 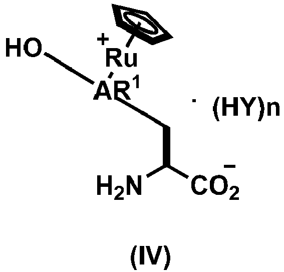 wherein AR¹ is an aromatic or heteroaromatic hydrocarbon having 5 to 14 carbon atoms, preferably six to ten carbon atoms, which may be further substituted by at least one C₁ to C₆ alkyl group and/or at least one heteroatom, Y is an anion, preferably selected from CF₃CO₂⁻, triflate⁻ or OH⁻, and n is 0 or 1.
4. A [AR¹(RuCp)-OH] complex having the general formula (IV) as defined in claim 3, wherein AR¹ is phenyl, 4-hydroxy-phenyl- or 1*H*-indol-3-yl, Y is an anion, preferably selected from CF₃CO₂⁻ , triflate⁻ or OH⁻, and n is 0 or 1.
5. Use of the [P-AR¹⁻(RuCp)-OH]⁻complex (I) as defined in claims 1 or 2 in a solid phase peptide synthesis, wherein AR¹, Y, n and P are as defined in claims 1 or 2.
6. An oligo- or polypeptide having at least one amino acid residue of the formula (V) incorporated into the amino acid backbone, wherein AR¹ is as defined in claims 3 or 4. 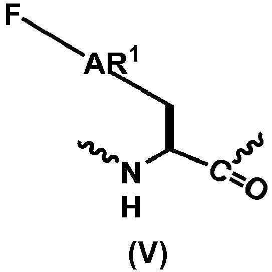
7. An oligo- or polypeptide having at least one amino acid residue of the formula (V) in the amino acid backbone, wherein AR¹ is phenyl; 4-hydroxy-phenyl- or 1*H*-indol-3-yl: 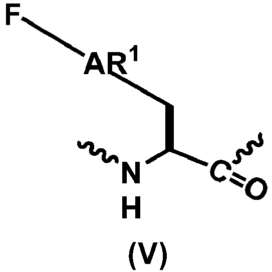
8. Use of an oligo- or polypeptide as defined in claim 6 or 7 for preparing a diagnostic composition for positron emission tomography (PET).
9. Process for preparing an oligo- or polypeptide of claim 7 having at least one amino acid residue of the formula (V) in the amino acid backbone, wherein AR¹ is phenyl; 4-hydroxy-phenyl- or 1*H*-indol-3-yl, 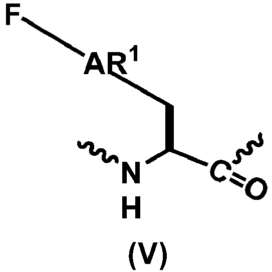 wherein an oligo- or polypeptide having at least one amino acid complex of the formula (IV) as defined in claims 3 or 4 incorporated into the amino acid backbone, wherein AR¹, Y and n are as defined in claims 3 or 4, is reacted with a fluorine source in the presence of an imidazolium chloride and an oxalate compound, selected from bis(trimethylneopentylammonium) oxalate or Kryptand 2.2.2 and alkali oxalate, to fluorinate the AR¹ compound, thereby providing an oligo- or polypeptide having a fluorinated AR¹compound as illustrated in the following scheme:

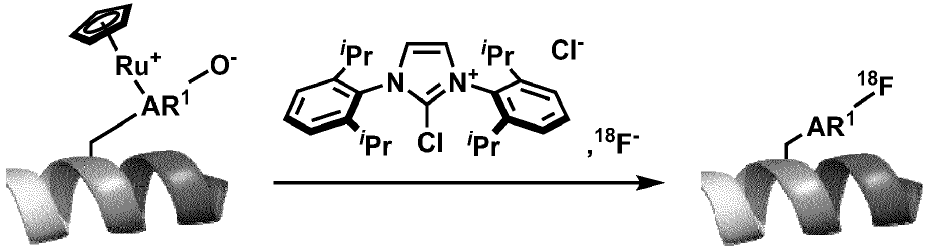

## Claims

1. A [P-AR¹(RuCp)-OH] complex having the general formula (I): 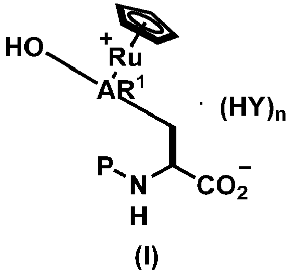
wherein AR¹ is an aromatic or heteroaromatic hydrocarbon having 5 to 14 carbon atoms, preferably six to ten carbon atoms, which may be further substituted by at least one C₁ to C₆ alkyl group and/or by at least one heteroatom,
wherein Y is an anion, preferably selected from CF₃CO₂⁻, triflate⁻ or OH⁻, n is 0 or 1, and P is a protective group, preferably a fluorenyl methoxy carbonyl (Fmoc), *tert-*butoxycarbonyl (Boc), or benzyloxycarbonyl (Cbz) group.

2. A [P-AR¹(RuCp)-OH] complex having the general formula (I) as defined in claim 1, wherein AR¹ is phenyl, 4-hydroxy-phenyl- or 1*H*-indol-3-yl, and Y, n and P have the meaning as defined in claim 1.

3. A [AR¹(RuCp)-OH] complex having the general formula (IV): 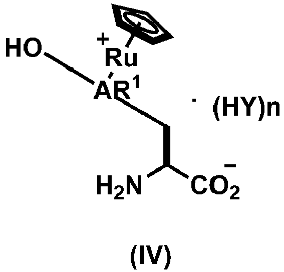 wherein AR¹ is an aromatic or heteroaromatic hydrocarbon having 5 to 14 carbon atoms, preferably six to ten carbon atoms, which may be further substituted by at least one C₁ to C₆ alkyl group and/or at least one heteroatom, Y is an anion, preferably selected from CF₃CO₂⁻, triflate⁻ or OH⁻, and n is 0 or 1.

4. A [AR¹(RuCp)-OH] complex having the general formula (IV) as defined in claim 3, wherein AR¹ is phenyl, 4-hydroxy-phenyl- or 1*H*-indol-3-yl, Y is an anion, preferably selected from CF₃CO₂⁻, triflate⁻ or OH⁻, and n is 0 or 1.

5. Use of the [P-AR¹⁻(RuCp)-OH]⁻complex (I) as defined in claims 1 or 2 in a solid phase peptide synthesis, wherein AR¹, Y, n and P are as defined in claims 1 or 2.

6. An oligo- or polypeptide having at least one amino acid residue of the formula (V) incorporated into the amino acid backbone, wherein AR¹ is as defined in claims 3 or 4.

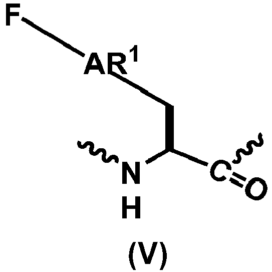

7. An oligo- or polypeptide having at least one amino acid residue of the formula (V) in the amino acid backbone, wherein AR¹ is phenyl; 4-hydroxy-phenyl- or 1*H*-indol-3-yl:

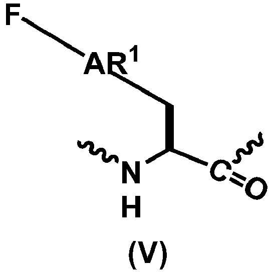

8. Use of an oligo- or polypeptide as defined in claim 6 or 7 for preparing a diagnostic composition for positron emission tomography (PET).

9. Process for preparing an oligo- or polypeptide of claim 7 having at least one amino acid residue of the formula (V) in the amino acid backbone, wherein AR¹ is phenyl; 4-hydroxy-phenyl- or 1*H*-indol-3-yl, 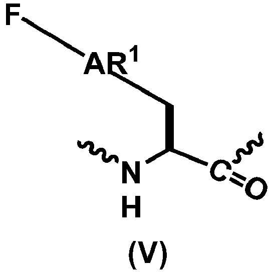 wherein an oligo- or polypeptide having at least one amino acid complex of the formula (IV) as defined in claims 3 or 4 incorporated into the amino acid backbone, wherein AR¹, Y and n are as defined in claims 3 or 4, is reacted with a fluorine source in the presence of an imidazolium chloride and an oxalate compound, selected from bis(trimethylneopentylammonium) oxalate or Kryptand 2.2.2 and alkali oxalate, to fluorinate the AR¹ compound, thereby providing an oligo- or polypeptide having a fluorinated AR¹compound as illustrated in the following scheme:
